# EUROPÄISCHE PATENTANMELDUNG

(11) **EP 2 292 608 A1**
(43) Veröffentlichungstag der Anmeldung: **09.03.2011**
(21) Anmeldenummer: 10182907.5
(22) Anmeldetag: 02.03.1998
(51) Int. Cl.: C07D 239/52, A01N 43/50, C07D 239/545, C07D 239/36, C07D 239/34, C07D 239/30

(54) **Zwischenprodukte zur Herstellung von substituierten Aminosalicylsäureamiden mit funizider Wirkung**

(30) Priorität: 14.03.1997 DE 19710609
(62) Teilanmeldung aus: 05002366.2
(71) Anmelder: Bayer CropScience AG, 40789 Monheim (DE)
(72) Erfinder: Seitz, Thomas Dr., 40764 Langenfeld (DE); Stelzer, Uwe Dr., 51399 Burscheid (DE); Wolfrum, Peter Dr., 40789 Monheim (DE); Stenzel, Klaus Dr., 40595 Düsseldorf (DE)

(57) **Zusammenfassung**

Die Erfindung betrifft neue substituierte Aminosalicylsäureamide, mehrere Verfahren zu ihrer Herstellung und ihre Verwendung als Fungizide, sowie neue Zwischenprodukte und mehrere Verfahren zu deren Herstellung.

## Beschreibung

Die Erfindung betrifft neue substituierte Aminosalicylsäureamide, mehrere Verfahren zu ihrer Herstellung und ihre Verwendung als Fungizide, sowie neue Zwischenprodukte und mehrere Verfahren zu deren Herstellung.

Bestimmte substituierte Acylaminosalicylsäureamide, wie beispielsweise die Verbindungen 3-Formamido-salicylanilid und 3-(Formylamino)-2-hydroxy-N-(phenyl-methyl)-benzamid, sind bereits bekannt (vergleiche z. B. Biochim. Biophys. Acta (1993), 1142(3), 262-8, J. Med. Chem. (1990), 33(1), 136-42 oder J. Biol. Chem. (1971), 246(23), 7125-30). Eine Wirkung gegen Schädlinge ist von diesen vorbekannten Verbindungen bisher jedoch nicht beschrieben.

Es wurden nun die neuen substituierten Aminosalicylsäureamide der allgemeinen Formel (I) gefunden, 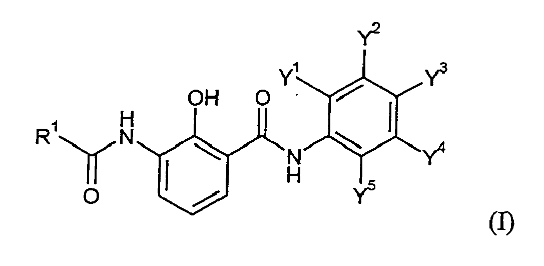 in welcher
- R¹: für Wasserstoff, Alkyl oder Alkoxy steht,
- Y¹, Y², Y³, Y⁴ und Y⁵: gleich oder verschieden sind und für Wasserstoff, Halogen, Cyano, Alkyl, Halogenalkyl, Alkoxy oder Halogenalkoxy stehen, und ent- weder
- Y² oder Y³: für -G-Z steht,
worin
G für eine der nachstehenden Gruppierungen -Q-CQ-, -CQ-Q-, -CQ-Q-CH₂-, -CH₂-Q-CQ-, -Q-CQ-CH₂-, -Q-CQ-Q-CH₂-, -S(O)ₙ-, -CH₂-S(O)ₙ-, -CQ-, -S(O)ₙ-CH₂-, -C(R³)=N-O-, -C(R³)=N-O-CH₂-, -N(R⁴)-, -CQ-N(R⁴)-, -N(R⁴)-CQ-, -Q-CQ-N(R⁴)-, -N=C(R³)-Q-CH₂-, -CH₂-O-N=C(R³)-, -C(CH3)-O-N=C(R³)-, -N(R⁴)-CQ-Q-, -CQ-N(R⁴)-CQ-Q-, -N(R⁴)-CQ-Q-CH₂-, -Q-C(R³)=N-O-CH₂-, -N(R⁴)-C(R³)=N-O-CH₂-,-O-CH₂-C(R³)=N-O-CH₂-, -N=N-C(R³)=N-O-CH₂-,-C(=N-O-R⁵)-C(R³)=N-O-CH₂-, -C(=N-O-R⁵)-C(R³)-O-N=CH-,-C(=N-O-R⁵)-C(R³)-O-N=C(CH₃)-, -T-Ar¹- oder -T-Ar¹-Q- steht,
wobei
Ar¹ für gegebenenfalls substituiertes Arylen, Heteroarylen, Cyclo- alkylen oder Heterocycloalkylen (d.h. ein zweifach verknüpfter aliphatischer Ring, in dem ein oder mehrere Kohlenstoffatome durch Heteroatome, d.h. von Kohlenstoff verschiedene Atome ersetzt sind) steht,
n für die Zahlen 0, 1 oder 2 steht,
Q für Sauerstoff oder Schwefel steht,
R³ für Wasserstoff, Cyano oder jeweils gegebenenfalls substituier- tes Alkyl, Alkoxy, Alkylthio, Alkylamino, Dialkylamino oder Cycloalkyl steht, und
R⁴ für Wasserstoff, Hydroxy, Cyano oder jeweils gegebenenfalls substituiertes Alkyl, Alkoxy oder Cycloalkyl steht,
R⁵ für Wasserstoff oder Alkyl steht und
T für eine Einfachbindung, für Sauerstoff Schwefel, -CH₂-O-, -CH₂-S- oder für gegebenenfalls substituiertes Alkandiyl steht,
Z für jeweils gegebenenfalls substituiertes Alkyl, Alkenyl, Alkinyl, Cyclo- alkyl, Aryl oder Heterocyclyl steht.

In den Definitionen sind die Kohlenwasserstoffketten, wie Alkyl, Alkylen, Alkenyl oder Alkinyl, auch in Verknüpfung mit Heteroatomen, wie in Alkoxy, Alkylthio oder Alkylamino, jeweils geradkettig oder verzweigt.

Halogen steht im allgemeinen für Fluor, Chlor, Brom oder Iod, vorzugsweise für Fluor, Chlor oder Brom, insbesondere für Fluor oder Chlor.

Aryl steht für aromatische, mono oder polycyclische Kohlenwasserstoffringe, wie z.B. Phenyl, Naphthyl, Anthranyl, Phenanthryl, vorzugsweise für Phenyl oder Naphthyl, insbesondere für Phenyl.

Heterocyclyl steht für gesättigte oder ungesättigte, sowie aromatische, ringförmige Verbindungen mit bis zu acht Ringgliedern, in denen mindestens ein Ringglied ein Heteroatom, d. h. ein von Kohlenstoff verschiedenes Atom, ist. Enthält der Ring mehrere Heteroatome, können diese gleich oder verschieden sein. Heteroatome sind bevorzugt Sauerstoff, Stickstoff oder Schwefel. Gegebenenfalls bilden die ringförmigen Verbindungen mit weiteren carbocyclischen oder heterocyclischen, ankondensierten oder überbrückten Ringen gemeinsam ein polycyclisches Ringsystem. Bevorzugt sind mono- oder bicyclische Ringsysteme, insbesondere mono- oder bicyclische, aromatische Ringsysteme.

Cycloalkyl steht für gesättigte, carbocyclische, ringförmige Verbindungen, die gegebenenfalls mit weiteren carbocyclischen, ankondensierten oder überbrückten Ringen ein polycyclisches Ringsystem bilden.

Cycloalkenyl steht für carbocyclische, ringförmige Verbindungen, die mindestens eine Doppelbindung enthalten und gegebenenfalls mit weiteren carbocyclischen, ankondensierten oder überbrückten Ringen ein polycyclisches Ringsystem bilden.

Weiterhin wurde gefunden, daß man die neuen substituierten Acylaminosalicylsäureamide der allgemeinen Formel (I) erhält, wenn man
a) Aminosalicylsäureamide der allgemeinen Formel (II), 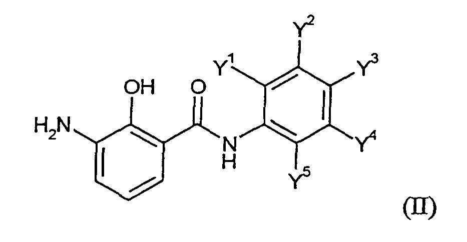 in welcher
   Y¹, Y², Y³, Y⁴ und Y⁵ die oben angegebenen Bedeutungen haben,
   mit Acylierungsmitteln der allgemeinen Formel (III), 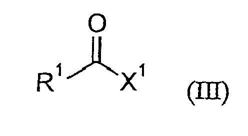 in welcher
   - R¹: die oben angegebene Bedeutung hat und
   - X¹: für Halogen, Hydroxy, Alkoxy oder Alkylcarbonyloxy steht,
   gegebenenfalls in Gegenwart eines Verdünnungsmittels, gegebenenfalls in Gegenwart eines Säureakzeptors, und gegebenenfalls in Gegenwart eines weiteren Reaktionshilfsmittels, umsetzt, oder wenn man
b) Nitrosalicylsäureamide der allgemeinen Formel (IV) 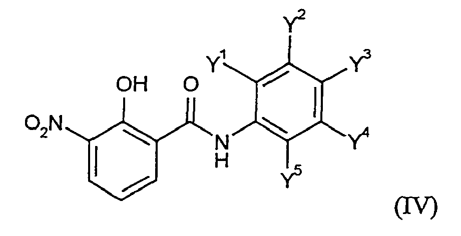 in welcher
   Y¹, Y², Y³, Y⁴ und Y⁵ die oben angegebenen Bedeutungen haben,
   mit Ameisensäure, gegebenenfalls in Gegenwart eines Katalysators und gegebenenfalls in Gegenwart eines weiteren Reaktionshilfsmittels, umsetzt, oder wenn man
c) O-Benzyl-nitrosalicylsäureamide der allgemeinen Formel (V), 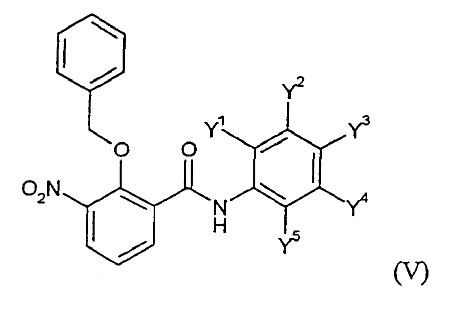 in welcher
   Y¹, Y², Y³, Y⁴ und Y⁵ die oben angegebenen Bedeutungen haben,
   mit Ameisensäure, gegebenenfalls in Gegenwart von Wasserstoff oder eines unedlen Metalls, gegebenenfalls in Gegenwart eines Katalysators und gegebenenfalls in Gegenwart eines weiteren Reaktionshilfsmittels, umsetzt.

Schließlich wurde gefunden, daß die neuen substituierten Aminosalicylsäureamide der allgemeinen Formel (I) eine sehr starke fungizide Wirkung zeigen.

Die erfindungsgemäßen Verbindungen und deren Vorprodukte liegen gegebenenfalls als Mischungen verschiedener möglicher isomerer Formen, insbesondere von Stereoisomeren, wie z. B. E- und Z-, threo- und erythro-, sowie optischen Isomeren oder auch Tautomeren vor. Es werden sowohl die E- als auch die Z-Isomeren, wie auch die threo- und erythro-, sowie die optischen Isomeren, als auch die möglichen Tautomeren, sowie beliebige Mischungen dieser Isomeren beansprucht.

In der allgemeinen Formel (I) steht
- R¹: vorzugsweise für Wasserstoff, Alkyl mit 1 bis 4 Kohlenstoffatomen oder Alkoxy mit 1 bis 4 Kohlenstoffatomen;
insbesondere für Wasserstoff, Methyl, Ethyl, n- oder i-Propyl, Methoxy oder Ethoxy; bevorzugt für Wasserstoff, Methyl oder Methoxy; besonders bevor- zugt für Wasserstoff.

In der allgemeinen Formel (I) stehen
- Y¹, Y⁴ und Y⁵: unabhängig voneinander vorzugsweise für Wasserstoff, Halogen, Cyano, Alkyl oder Alkoxy mit jeweils 1 bis 4 Kohlenstoffatomen, Halogen- alkyl, oder Halogenalkoxy, mit jeweils 1 bis 4 Kohlenstoffatomen; insbesondere für Wasserstoff, Fluor, Chlor, Brom, Cyano, Methyl, Ethyl, Methoxy, Trifluormethyl oder Difluormethoxy; bevorzugt für Wasserstoff.

In der allgemeinen Formel (I) stehen
- Y² und Y³: unabhängig voneinander vorzugsweise für Wasserstoff, Halogen, Cyano, Alkyl oder Alkoxy mit jeweils 1 bis 4 Kohlenstoffatomen, Halogenalkyl, oder Halogenalkoxy mit jeweils 1 bis 4 Kohlenstoffatomen und 1 bis 5 gleichen oder verschiedenen Halogenatomen und einer der Reste Y² oder Y³ für die Gruppe -G-Z.

Insbesondere seht Y² für Wasserstoff, Fluor, Chlor, Brom, Cyano, Methyl, Ethyl, Methoxy, Trifluormethyl oder Difluormethoxy; bevorzugt für Wasserstoff.

Insbesondere seht Y³ für Wasserstoff, Fluor, Chlor, Brom, Cyano, Methyl, Ethyl, Methoxy, Trifluormethyl oder Difluormethoxy; bevorzugt für Gruppe -G-Z.

In der allgemeinen Formel (I) steht G vorzugsweise für eine der nachstehenden Gruppierungen

-Q-CQ-, -CQ-Q-, -CQ-Q-CH₂-, -CH₂-Q-CQ-, -Q-CQ-CH₂-, -Q-CQ-Q-CH₂-, -N=N-, -S(O)ₙ-, -CH₂-S(O)ₙ-, -CQ-, -S(O)n-CH₂-, -C(R³)=N-O-, -C(R³)=N-O-CH₂-, -N(R⁴)-, -CQ-N(R⁴)-, -N(R⁴)-CQ-, -Q-CQ-N(R⁴)-, -N=C(R³)-Q-CH₂-, -CH₂-O-N=C(R³)-, -C(CH₃)-O-N=C(R³)-, -N(R⁴)-CQ-Q-, -CQ-N(R⁴)-CQ-Q-,-N(R⁴)-CQ-Q-CH₂-, -Q-C(R³)=N-O-CH₂-, -N(R⁴)-C(R³)=N-O-CH₂-, -O-CH₂-C(R³)=N-O-CH₂-, -N=N-C(R³)=N-O-CH₂-, -C(=N-O-R⁵)-C(R³)=N-O-CH₂-, -C(=N-O-R⁵)-C(R³)-O-N=CH-,-C(=N-O-R⁵)-C(R³)-O-N=C(CH₃)-, -T-Ar¹- oder -T-Ar¹-Q-;
wobei Q, n, R³, R⁴ und R⁵ die in der Anmeldung angegebenen Bedeutungen haben.

Insbesondere steht G für -C(R³) = N-O-CH₂-, wobei R³ vorzugsweise für Cyclopropyl und insbesondere für Methyl steht.

Bevorzugt steht G für -T-Ar¹-, -T-Ar¹-S- und insbesondere für -T-Ar¹-O-, wobei
- T: vorzugsweise für eine Einfachbindung, für Sauerstoff, Schwefel, -CH₂-O-, -CH₂-S- oder für Alkandiyl mit 1 bis 3 Kohlenstoffatomen, insbesondere für eine Einfachbindung, für Sauerstoff, Schwefel, -CH₂-O-, -CH₂-S-, Methylen, Ethylen oder Propylen und bevorzugt für Sauerstoff steht.
- Ar¹: steht vorzugsweise für jeweils gegebenenfalls einfach oder mehrfach, gleich oder verschieden substituiertes Phenylen, Naphthylen, Cycloalkylen oder für Heteroarylen oder Heterocycloalkylen mit 3 bis 7 Ringgliedern, von denen mindestens eines für Sauerstoff, Schwefel oder Stickstoff und gegebenenfalls ein oder zwei weitere für Stickstoff stehen, steht, wobei die möglichen Sub- stituenten vorzugsweise aus der nachstehenden Aufzählung ausgewählt sind: Halogen, Cyano, Amino, Hydroxy, Formyl, Carboxy, Carbamoyl, Thiocarb- amoyl;
jeweils geradkettiges oder verzweigtes Alkyl, Alkoxy, Alkylthio, Alkylsulfinyl oder Alkylsulfonyl mit jeweils 1 bis 6 Kohlenstoffatomen;
jeweils geradkettiges oder verzweigtes Alkenyl oder Alkenyloxy mit jeweils 2 bis 6 Kohlenstoffatomen;
jeweils geradkettiges oder verzweigtes Halogenalkyl, Halogenalkoxy, Halo- genalkylthio, Halogenalkylsulfinyl oder Halogenalkylsulfonyl mit jeweils 1 bis 6 Kohlenstoffatomen und 1 bis 13 gleichen oder verschiedenen Halogen- atomen;
jeweils geradkettiges oder verzweigtes Halogenalkenyl oder Halogenalkenyl- oxy mit jeweils 2 bis 6 Kohlenstoffatomen und 1 bis 11 gleichen oder ver- schiedenen Halogenatomen;
jeweils geradkettiges oder verzweigtes Alkylamino, Dialkylamino, Alkyl- carbonyl, Alkylcarbonyloxy, Alkoxycarbonyl, Alkylsulfonyloxy, Hydroximino- alkyl oder Alkoximinoalkyl mit jeweils 1 bis 6 Kohlenstoffatomen in den ein- zelnen Alkylteilen, sowie
Cycloalkyl mit 3 bis 6 Kohlenstoffatomen und
- Ar¹: steht insbesondere
für jeweils gegebenenfalls einfach bis dreifach, gleich oder verschieden sub- stituiertes Phenylen oder Pyridindiyl, für jeweils gegebenenfalls einfach sub- stituiertes Pyrimidindiyl, Pyridazindiyl, Pyrazindiyl, 1,2,3-Triazindiyl, 1,2,4- Triazindiyl oder 1,3,5-Triazindiyl oder für 1,2,4-Thiadiazoldiyl, 1,3,4-Thia- diazoldiyl, 1,2,4-Oxadiazoldiyl, 1,3,4-Oxadiazoldiyl steht, wobei die mög- lichen Substituenten vorzugsweise aus der nachstehenden Aufzählung ausge- wählt sind:
Fluor, Chlor, Brom, Cyano, Methyl, Ethyl, n odor i Propyl, n , i , o odor t-Butyl, Cyclopropyl, Methoxy, Ethoxy, n- oder i-Propoxy, Methylthio, Ethyl- thio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Trifluormethylthio, Difluorchlormethylthio, Trifluormethylsulfinyl oder Trifluormethylsulfonyl und
- Ar¹: steht bevorzugt
für 1,3,4-Thiadiazoldiyl, 1,2,4-Oxadiazoldiyl, 1,3,4-Oxadiazoldiyl oder für jeweils gegebenenfalls einfach oder zweifach, gleich oder verschieden substitu- iertes Pyridindiyl, Pyrimidindiyl oder 1,3,5-Triazindiyl, wobei die oben angege- benen Substituenten in Frage kommen oder besonders bevorzugt für 1,2,4-Thiadiazoldiyl oder gegebenenfalls einfach oder zweifach, gleich oder verschieden substituiertes Pyrimidindiyl steht, wobei die oben angegebenen Substituenten in Frage kommen und die Substituenten vorzugsweise für Halogen, insbesondere für Fluor stehen.

In der allgemeinen Formel (I) steht
- Z: vorzugsweise für gegebenenfalls einfach oder mehrfach, gleich oder verschieden durch Halogen, Cyano, Hydroxy, Amino, C₁-C₄-Alkoxy, C₁-C₄- Alkylthio, C₁-C₄-Alkylsulfinyl oder C₁-C₄-Alkylsulfonyl (welche jeweils ge- gebenenfalls durch Halogen substituiert sein können) substituiertes Alkyl mit 1 bis 8 Kohlenstoffatomen;
für jeweils gegebenenfalls durch Halogen substituiertes Alkenyl oder Alkinyl mit jeweils bis zu 8 Kohlenstoffatomen;
für jeweils gegebenenfalls einfach oder mehrfach, gleich oder verschieden durch Halogen, Cyano, Carboxy, Phenyl (welches gegebenenfalls durch Halo- gen, Cyano, C₁-C₄-Alkyl, C₁-C₄-Halogenalkyl, C₁-C₄-Alkoxy oder C₁-C₄- Halogenalkoxy substituiert ist), C₁-C₄-Alkyl oder C₁-C₄-Alkoxy-carbonyl substiuiertes Cycloalkyl mit 3 bis 6 Kohlenstoffatomen;
für jeweils gegebenenfalls einfach oder mehrfach, gleich oder verschieden sub- stituiertes Phenyl, Naphthyl oder für Heterocyclyl mit 3 bis 7 Ringgliedern, von denen mindestens eines für Sauerstoff, Schwefel oder Stickstoff und gege- benenfalls ein oder zwei weitere für Stickstoff stehen, steht, wobei die mög- lichen Substituenten vorzugsweise aus der nachstehenden Aufzählung ausge- wählt sind:
Halogen, Cyano, Amino, Hydroxy, Formyl, Carboxy, Carbamoyl, Thiocarb- amoyl;
jeweils geradkettiges oder verzweigtes Alkyl, Alkoxy, Alkylthio, Alkylsulfinyl oder Alkylsulfonyl mit jeweils 1 bis 6 Kohlenstoffatomen;
jeweils geradkettiges oder verzweigtes Alkenyl oder Alkenyloxy mit jeweils 2 bis 6 Kohlenstoffatomen;
jeweils geradkettiges oder verzweigtes Halogenalkyl, Halogenalkoxy, Halo- genalkylthio, Halogenalkylsulfinyl oder Halogenalkylsulfonyl mit jeweils 1 bis 6 Kohlenstoffatomen und 1 bis 13 gleichen oder verschiedenen Halogen- atomen;
jeweils geradkettiges oder verzweigtes Halogenalkenyl oder Halogenalkenyl- oxy mit jeweils 2 bis 6 Kohlenstoffatomen und 1 bis 11 gleichen oder verschie- denen Halogenatomen;
jeweils geradkettiges oder verzweigtes Alkylamino, Dialkylamino, Alkyl- carbonyl, Alkylcarbonyloxy, Alkoxycarbonyl oder Alkylsulfonyloxy, mit je- weils 1 bis 6 Kohlenstoffatomen in den einzelnen Alkylteilen;
jeweils gegebenenfalls einfach oder mehrfach, gleich oder verschieden durch Halogen und/oder geradkettiges oder verzweigtes Alkyl mit 1 bis 4 Kohlen- stoffatomen und/oder geradkettiges oder verzweigtes Halogenalkyl mit 1 bis 4 Kohlenstoffatomen und 1 bis 9 gleichen oder verschiedenen Halogenatomen substituiertes, jeweils zweifach verknüpftes Alkylen oder Dioxyalkylen mit jeweils 1 bis 6 Kohlenstoffatomen;
Cycloalkyl mit 3 bis 6 Kohlenstoffatomen;
jeweils gegebenenfalls einfach oder mehrfach, gleich oder verschieden durch Halogen und/oder geradkettiges oder verzweigtes Alkyl mit 1 bis 4 Kohlen- stoffatomen substituiertes Phenyl oder Phenoxy;
Heterocyclyl oder Heterocyclyl-methyl mit jeweils 3 bis 7 Ringgliedern, von denen jeweils 1 bis 3 gleiche oder verschiedene Heteroatome sind - insbeson- dere Stickstoff, Sauerstoff und/oder Schwefel-, oder
eine Gruppierung 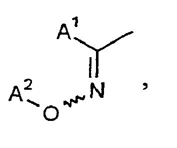 worin
A¹ für Alkyl mit 1 bis 4 Kohlenstoffatomen oder Cycloalkyl mit 1 bis 6 Kohlenstoffatomen steht und
A² für gegebenenfalls durch Cyano, Alkoxy, Alkylthio, Alkylamino, Dialkylamino oder Phenyl substituiertes Alkyl mit 1 bis 4 Kohlenstoff- atomen, Alkenyl oder Alkinyl mit jeweils 2 bis 4 Kohlenstoffatomen steht, oder eine Gruppierung 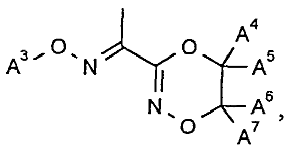 in welcher
A³ für Alkyl mit 1 bis 4 Kohlenstoffatomen steht und
A⁴, A⁵, A⁶ und A⁷ gleich oder verschieden sind und unabhängig voneinander für Wasserstoff Alkyl oder Hydroxyalkyl mit jeweils 1 bis 4 Kohlen- stoffatomen oder Halogenalkyl mit 1 bis 4 Kohlenstoffatomen und 1 bis fünf gleichen oder verschiedenen Halogenatomen stehen, oder
A⁴ und A⁵ oder A⁴ und A⁶ oder A⁶ und A⁷ gemeinsam mit den jeweiligen Kohlenstoffatomen, an die sie gebunden sind, einen cycloaliphatischen Ring mit fünf, sechs oder sieben Kohlenstoffatomen bilden.

In der allgemeinen Formel (I) steht
- Z: insbesondere für gegebenenfalls einfach bis dreifach, gleich oder verschieden durch Fluor, Chlor, Cyano oder Methoxy substituiertes Methyl, Ethyl, n- oder i-Propyl, n-, i-, s- oder t-Butyl;
für jeweils gegebenenfalls durch Fluor oder Chlor substituiertes Vinyl, Allyl oder Propargyl;
für jeweils gegebenenfalls einfach bis dreifach, gleich oder verschieden durch Fluor, Chlor, Cyano, Methoxy, Phenyl, Methyl oder Ethyl substiuiertes Cyclo- propyl, Cyclobutyl, Cyclopentyl oder Cyclohexyl;
und bevorzugt für jeweils gegebenenfalls einfach bis dreifach, gleich oder verschieden substituiertes Phenyl, 1,2,4-Thiadiazolyl, 1,3,4-Thiadiazolyl, 1,2,4-Oxadiazolyl, 1,3,4-Oxadiazolyl, Pyridinyl, Pyrimidyl, Pyridazinyl, Pyrazinyl, 1,2,3-Triazinyl, 1,2,4-Triazinyl oder 1,3,5-Triazinyl steht, wobei die möglichen Substituenten die oben angegebenen Bedeutungen haben und vor- zugsweise aus der nachstehenden Aufzählung ausgewählt sind:
Fluor, Chlor, Brom, Cyano, Methyl, Ethyl, n- oder i-Propyl, n-, i-, s- oder t- Butyl, Methoxy, Ethoxy, n- oder i-Propoxy, Methylthio, Ethylthio, n- oder i- Propylthio, Methylsulfinyl, Ethylsulfinyl, Methylsulfonyl oder Ethylsulfonyl, Trifluormethyl, Trifluorethyl, Difluormethoxy, Trifluormethoxy, Difluor- chlormethoxy, Trifluorethoxy, Difluormethylthio, Trifluormethylthio, Difluor- chlormethylthio, Trifluormethylsulfinyl oder Trifluormethylsulfonyl, Methoxy- carbonyl, Ethoxycarbonyl,
jeweils gegebenenfalls einfach bis vierfach, gleich oder verschieden durch Fluor, Chlor, Methyl, Trifluormethyl oder Ethyl substituiertes, jeweils zweifach verknüpftes Methylendioxy, Ethylendioxy,
Phenyl, 4-Chlorphenyl, 4-Methylphenyl, Phenoxy, 4-Chlorphenoxy, 4- Methylphenoxy, Cyclopropyl, Cyclobutyl, Cyclopentyl oder Cyclohexyl, oder
eine Gruppierung 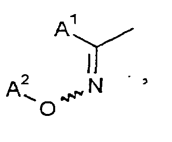 worin
A¹ für Methyl, Ethyl, n- oder i-Propyl, n-, i-, s- oder t-Butyl, Cyclopropyl oder Cyclobutyl steht,
A² für Methyl, Ethyl, n- oder i-Propyl, n-, i-, s- oder t-Butyl, Allyl, Propargyl, But-2-en-1-yl, 2-Methyl-prop-1-en-3-yl, Cyanmethyl, Methoxymethyl, Ethoxymethyl, Methoxyethyl, Ethoxyethyl, Methylthiomethyl, Ethylthiomethyl, Methylthioethyl, Ethylthioethyl, Dimethylaminomethyl, Dimethylaminoethyl, Methylaminomethyl, Methylaminoethyl oder Benzyl steht, oder eine Gruppierung 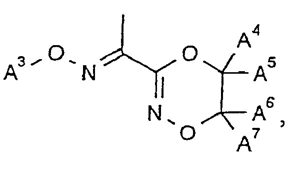 in welcher
A³ für Methyl oder Ethyl steht und
A⁴, A⁵, A⁶ und A⁷ gleich oder verschieden sind und unabhängig voneinander für Wasserstoff, Methyl, Ethyl, n- oder i-Propyl, n-, i-, s- oder t-Butyl, Hydroxymethyl, Trifluormethyl oder Trifluorethyl stehen, oder
A⁴ und A⁵ oder A⁴ und A⁶ oder A⁶ und A⁷ gemeinsam mit den jeweiligen Kohlenstoffatomen, an die sie gebunden sind, einen cycloaliphatischen Ring mit fünf, sechs oder sieben Kohlenstoffatomen bilden.

In der allgemeinen Formel (I) steht
- Z: bevorzugt für gegebenenfalls einfach bis dreifach, gleich oder verschieden durch Fluor, Chlor, Cyano oder Methoxy substituiertes Methyl, Ethyl, n- oder i-Propyl, n-, i-, S- oder t-Butyl;
für jeweils gegebenenfalls durch Fluor oder Chlor substituiertes Vinyl, Allyl oder Propargyl;
für jeweils gegebenenfalls einfach bis dreifach, gleich oder verschieden durch Fluor, Chlor, Cyano, Methoxy, Phenyl, Methyl oder Ethyl substituiertes Cyclopropyl, Cyclobutyl, Cyclopentyl oder Cyclohexyl;
und bevorzugt für jeweils gegebenenfalls einfach bis dreifach, gleich oder verschieden substituiertes Phenyl, Pyridyl, Pyrimidyl oder Thienyl, wobei die möglichen Substituenten die oben angegebenen Bedeutungen haben und vor- zugsweise aus der nachstehenden Aufzählung ausgewählt sind:
Fluor, Chlor, Brom, Cyano, Methyl, Ethyl, n- oder i-Propyl, n-, i-, s- oder t- Butyl, Methoxy, Ethoxy, n- oder i-Propoxy, Methylthio, Ethylthio, n- oder i- Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlor- methoxy, Trifluorethoxy, Difluormethylthio, Trifluormethylthio, Difluorchlor- methylthio, Trifluormethylsulfinyl, Trifluormethylsulfonyl, Methoxycarbonyl, Ethoxycarbonyl oder Phenoxy;
jeweils gegebenenfalls einfach bis vierfach, gleich oder verschieden durch Fluor, Chlor, Methyl, Trifluormethyl oder Ethyl substituiertes, jeweils zwei- fach verknüpftes Methylendioxy, Ethylendioxy;
Cyclopropyl, Cyclobutyl, Cyclopentyl, Cyclohexyl, Phenyl, Phenoxy oder
eine Gruppierung 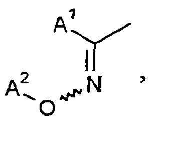 worin
A¹ für Methyl, Ethyl, n- oder i-Propyl, n-, i-, s- oder t-Butyl, Cyclopropyl oder Cyclobutyl steht,
A² für Methyl, Ethyl, n- oder i-Propyl, n-, i-, s- oder t-Butyl, Allyl, Propargyl, But-2-en-1-yl, 2-Methyl-prop-1-en-3-yl, Cyanmethyl, Methoxymethyl, Ethoxymethyl, Methoxyethyl, Ethoxyethyl, Methylthiomethyl, Ethylthiomethyl, Methylthioethyl, Ethylthioethyl, Dimethylaminomethyl, Dimethylaminoethyl, Methylaminomethyl, Methylaminoethyl oder Benzyl steht, oder eine Gruppierung 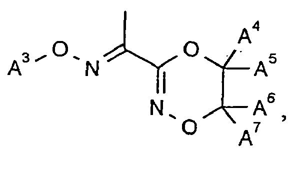 in welcher
A³ für Methyl oder Ethyl steht und
A⁴, A⁵, A⁶ und A⁷ gleich oder verschieden sind und unabhängig voneinander für Wasserstoff, Methyl, Ethyl, n- oder i-Propyl, n-, i-, s- oder t-Butyl, Hydroxymethyl, Trifluormethyl oder Trifluorethyl stehen, oder
A⁴ und A⁵ oder A⁴ und A⁶ oder A⁶ und A⁷ gemeinsam mit den jeweiligen Kohlenstoffatomen, an die sie gebunden sind, einen cycloaliphatischen Ring mit funf, sechs oder sieben Kohlenstoffatomen bilden.

In der allgemeinen Formel (I) steht
- Z: besonders bevorzugt für jeweils gegebenenfalls einfach bis dreifach, gleich oder verschieden substituiertes Pyridyl, Pyrimidyl und insbesondere für unsub- stituiertes oder einfach bis dreifach, gleich oder verschieden substituiertes Phenyl, wobei die möglichen Substituenten die oben angegebenen Bedeu- tungen haben und vorzugsweise aus der nachstehenden Aufzählung ausge- wählt sind:
Fluor, Chlor, Brom, Cyano, Methyl, Ethyl, n- oder i-Propyl, n-, i-, s- oder t- Butyl, Methoxy, Ethoxy, n- oder i-Propoxy, Methylthio, Ethylthio, n- oder i- Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlor- methoxy, Trifluorethoxy, Difluormethylthio, Trifluormethylthio, Difluorchlor- methylthio, Trifluormethylsulfinyl, Trifluormethylsulfonyl, Methoxycarbonyl, Ethoxycarbonyl oder Phenoxy;

Gegenstand der vorliegenden Anmeldung sind vorzugsweise Aminosalicylsäureamide der Formel (I),
in welcher
- R¹: für Wasserstoff, Alkyl mit 1 bis 4 Kohlenstoffatomen oder Alkoxy mit 1 bis 4 Kohlenstoffatomen steht,
- Y¹, Y², Y³, Y⁴ und Y⁵: gleich oder verschieden sind und für Wasserstoff, Halogen, Cyano, Alkyl oder Alkoxy mit jeweils 1 bis 4 Kohlenstoffatomen, Halogen- alkyl, oder Halogenalkoxy mit jeweils 1 bis 4 Kohlenstoffatomen und 1 bis 5 gleichen oder verschiedenen Halogenatomen stehen, und entweder
- Y² oder: Y³ für -G-Z steht,
worin
G für eine der nachstehenden Gruppierungen -Q-CQ-, -CQ-Q-, -CQ-Q-CH₂-, -CH₂-Q-CQ-, -Q-CQ-CH₂-, -Q-CQ-Q-CH₂-, -N=N-, -S(O)ₙ-, -CH₂-S(O)ₙ-, -CQ-, -S(O)ₙ-CH₂-, -C(R³)=N-O-, -C(R³)=N-O-CH₂-, -N(R⁴)-, -CQ-N(R⁴)-, -N(R⁴)-CQ-, -Q-CQ-N(R⁴)-, -N=C(R³)-Q-CH₂-, -CH₂-O-N=C(R³)-, -C(CH₃)-O-N=C(R³)-, -N(R⁴)-CQ-Q-, -CQ-N(R⁴)-CQ-Q-, -N(R⁴)-CQ-Q-CH₂-, -Q-C(R³)=N-O-CH₂-, -N(R⁴)-C(R³)=N-O-CH₂-, -O-CH₂-C(R³)=N-O-CH₂-, -N=N-C(R³)=N-O-CH₂-,-C(=N-O-R⁵)-C(R³)=N-O-CH₂-, -C(=N-O-R⁵)-C(R³)-O-N=CH-,-C(=N-O-R⁵)-C(R³)-O-N=C(CH₃)-, -T-Ar¹- oder -T-Ar¹-Q- steht,
wobei
n für die Zahlen 0, 1 oder 2 steht,
Q für Sauerstoff oder Schwefel steht,
R³ für Wasserstoff, Cyano, für jeweils gegebenenfalls durch Halo- gen, Cyano oder C₁-C₁-Alkoxy substituiertes Alkyl, Alkoxy, Alkylthio, Alkylamino oder Dialkylamino mit jeweils 1 bis 6 Kohlenstoffatomen in den Alkylgruppen oder für jeweils gege- benenfalls durch Halogen, Cyano, Carboxy, C₁-C₁-Alkyl oder C₁-C₁-Alkoxy-carbonyl substituiertes Cycloalkyl mit 3 bis 6 Kohlenstoffatomen steht, und
R⁴ für Wasserstoff, Hydroxy, Cyano oder für gegebenenfalls durch Halogen, Cyano oder C₁-C₁-Alkoxy substituiertes Alkyl mit 1 bis 6 Kohlenstoffatomen oder für gegebenenfalls durch Halo- gen, Cyano, Carboxy, C₁-C₄-Alkyl oder C₁-C₄-Alkoxy- carbonyl substituiertes Cycloalkyl mit 3 bis 6 Kohlenstoffato- men steht,
R⁵ für Wasserstoff oder Alkyl mit 1 bis 4 Kohlenstoffatomen steht,
Ar¹ für jeweils gegebenenfalls einfach oder mehrfach, gleich oder verschieden substituiertes Phenylen, Naphthylen, Cycloalkylen oder für Heteroarylen oder Heterocycloalkylen mit 3 bis 7 Ringgliedern, von denen mindestens eines für Sauerstoff, Schwefel oder Stickstoff und gegebenenfalls ein oder zwei weitere für Stickstoff stehen, steht, wobei die möglichen Sub- stituenten vorzugsweise aus der nachstehenden Aufzählung ausgewählt sind:
Halogen, Cyano, Amino, Hydroxy, Formyl, Carboxy, Carb- amoyl, Thiocarbarnoyl;
jeweils geradkettiges oder verzweigtes Alkyl, Alkoxy, Alkyl- thio, Alkylsulfinyl oder Alkylsulfonyl mit jeweils 1 bis 6 Koh- lenstoffatomen;
jeweils geradkettiges oder verzweigtes Alkenyl oder Alkenyl- oxy mit jeweils 2 bis 6 Kohlenstoffatomen;
jeweils geradkettiges oder verzweigtes Halogenalkyl, Halogen- alkoxy, Halogenalkylthio, Halogenalkylsulfinyl oder Halogen- alkylsulfonyl mit jeweils 1 bis 6 Kohlenstoffatomen und 1 bis 13 gleichen oder verschiedenen Halogenatomen;
jeweils geradkettiges oder verzweigtes Halogenalkenyl oder Halogenalkenyloxy mit jeweils 2 bis 6 Kohlenstoffatomen und 1 bis 11 gleichen oder verschiedenen Halogenatomen;
jeweils geradkettiges oder verzweigtes Alkylamino, Dialkyl- amino, Alkylcarbonyl, Alkylcarbonyloxy, Alkoxycarbonyl, Alkylsulfonyloxy, Hydroximinoalkyl oder Alkoximinoalkyl mit jeweils 1 bis 6 Kohlenstoffatomen in den einzelnen Alkylteilen, sowie
Cycloalkyl mit 3 bis 6 Kohlenstoffatomen und
T für eine Einfachbindung, für Sauerstoff Schwefel, -CH₂-O-, -CH₂-S- oder für Alkandiyl mit 1 bis 3 Kohlenstoffatomen steht,
- Z: für gegebenenfalls einfach oder mehrfach, gleich oder verschieden durch Halogen, Cyano, Hydroxy, Amino, C₁-C₄-Alkoxy, C₁-C₄-Alkylthio, C₁-C₄- Alkylsulfinyl oder C₁-C₄-Alkylsulfonyl (welche jeweils gegebenenfalls durch Halogen substituiert sein können) substituiertes Alkyl mit 1 bis 8 Kohlenstoff- atomen;
für jeweils gegebenenfalls durch Halogen substituiertes Alkenyl oder Alkinyl mit jeweils bis zu 8 Kohlenstoffatomen;
für jeweils gegebenenfalls einfach oder mehrfach, gleich oder verschieden durch Halogen, Cyano, Carboxy, Phenyl (welches gegebenenfalls durch Halo- gen, Cyano, C₁-C₄-Alkyl, C₁-C₄-Halogenalkyl, C₁-C₄-Alkoxy oder C₁-C₄- Halogenalkoxy substituiert ist), C₁-C₄-Alkyl oder C₁-C₄-Alkoxy-carbonyl substiuiertes Cycloalkyl mit 3 bis 6 Kohlenstoffatomen;
für jeweils gegebenenfalls einfach oder mehrfach, gleich oder verschieden sub- stituiertes Phenyl, Naphthyl oder für Heterocyclyl mit 3 bis 7 Ringgliedern, von denen mindestens eines für Sauerstoff, Schwefel oder Stickstoff und gege- benenfalls ein oder zwei weitere für Stickstoff stehen, steht, wobei die mög- lichen Substituenten vorzugsweise aus der nachstehenden Aufzählung ausge- wählt sind:
Halogen, Cyano, Amino, Hydroxy, Formyl, Carboxy, Carbamoyl, Thiocarb- amoyl;
jeweils geradkettiges oder verzweigtes Alkyl, Alkoxy, Alkylthio, Alkylsulfinyl oder Alkylsulfonyl mit jeweils 1 bis 6 Kohlenstoffatomen;
jeweils geradkettiges oder verzweigtes Alkenyl oder Alkenyloxy mit jeweils 2 bis 6 Kohlenstoffatomen;
jeweils geradkettiges oder verzweigtes Halogenalkyl, Halogenalkoxy, Halo- genalkylthio, Halogenalkylsulfinyl oder Halogenalkylsulfonyl mit jeweils 1 bis 6 Kohlenstoffatomen und 1 bis 13 gleichen oder verschiedenen Halogen- atomen;
jeweils geradkettiges oder verzweigtes Halogenalkenyl oder Halogenalkenyl- oxy mit jeweils 2 bis 6 Kohlenstoffatomen und 1 bis 11 gleichen oder verschie- denen Halogenatomen;
jeweils geradkettiges oder verzweigtes Alkylamino, Dialkylamino, Alkyl- carbonyl, Alkylcarbonyloxy, Alkoxycarbonyl oder Alkylsulfonyloxy, mit je- weils 1 bis 6 Kohlenstoffatomen in den einzelnen Alkylteilen;
jeweils gegebenenfalls einfach oder mehrfach, gleich oder verschieden durch Halogen und/oder geradkettiges oder verzweigtes Alkyl mit 1 bis 4 Kohlen- stoffatomen und/oder geradkettiges oder verzweigtes Halogenalkyl mit 1 bis 4 Kohlenstoffatomen und 1 bis 9 gleichen oder verschiedenen Halogenatomen substituiertes, jeweils zweifach verknüpftes Alkylen oder Dioxyalkylen mit jeweils 1 bis 6 Kohlenstoffatomen;
Cycloalkyl mit 3 bis 6 Kohlenstoffatomen;
jeweils gegebenenfalls einfach oder mehrfach, gleich oder verschieden durch Halogen und/oder geradkettiges oder verzweigtes Alkyl mit 1 bis 4 Kohlen- stoffatomen substituiertes Phenyl oder Phenoxy;
Heterocyclyl oder Heterocyclyl-methyl mit jeweils 3 bis 7 Ringgliedern, von denen jeweils 1 bis 3 gleiche oder verschiedene Heteroatome sind - insbeson- dere Stickstoff, Sauerstoff und/oder Schwefel-, oder
eine Gruppierung 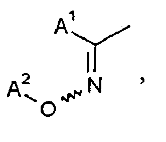 worin
A¹ für Alkyl mit 1 bis 4 Kohlenstoffatomen oder Cycloalkyl mit 1 bis 6 Kohlenstoffatomen steht und
A² für gegebenenfalls durch Cyano, Alkoxy, Alkylthio, Alkylamino, Dialkylamino oder Phenyl substituiertes Alkyl mit 1 bis 4 Kohlenstoff- atomen, Alkenyl oder Alkinyl mit jeweils 2 bis 4 Kohlenstoffatomen steht, oder eine Gruppierung 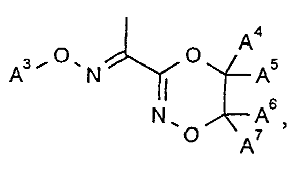 in welcher
A³ für Alkyl mit 1 bis 4 Kohlenstoffatomen steht und
A⁴, A⁵, A⁶ und A⁷ gleich oder verschieden sind und unabhängig voneinander für Wasserstoff, Alkyl oder Hydroxyalkyl mit jeweils 1 bis 4 Kohlen- stoffatomen oder Halogenalkyl mit 1 bis 4 Kohlenstoffatomen und 1 bis fünf gleichen oder verschiedenen Halogenatomen stehen, oder
A⁴ und A⁵ oder A⁴ und A⁶ oder A⁶ und A⁷ gemeinsam mit den jeweiligen Kohlenstoffatomen, an die sie gebunden sind, einen cycloaliphatischen Ring mit fünf, sechs oder sieben Kohlenstoffatomen bilden.

Die vorliegende Anmeldung betrifft insbesondere Verbindungen der Formel (I),
in welcher
- R¹: für Wasserstoff, Methyl, Ethyl, n- oder i-Propyl, Methoxy oder Ethoxy steht,
- Y¹, Y², Y³, Y⁴ und Y⁵: gleich oder verschieden sind und für Wasserstoff, Fluor, Chlor, Brom, Cyano, Methyl, Ethyl, Methoxy, Trifluormethyl oder Difluor- methoxy stehen, und entweder
- Y² oder Y³: für -G-Z steht,
worin
G für eine der nachstehenden Gruppierungen -Q-CQ-, -CQ-Q-, -CQ-Q-CH₂-, -CH₂-Q-CQ-, -Q-CQ-CH₂-, -Q-CQ-Q-CH₂-, -N=N-, -S(O)ₙ-, -CH₂-S(O)ₙ-, -CQ-, -S(O)ₙ-CH₂-, -C(R³)=N-O-, -C(R³)=N-O-CH₂-, -N(R⁴)-, -CQ-N(R⁴)-, -N(R⁴)-CQ-, -Q-CQ-N(R⁴)-, -N=C(R³)-Q-CH₂-, -CH₂-O-N=C(R³)-, -C(CH₃)-O-N=C(R³)-, -N(R⁴)-CQ-Q-, -CQ-N(R⁴)-CQ-Q-, -N(R⁴)-CQ-Q-CH₂-, -Q-C(R³)=N-O-CH₂-, -N(R⁴)-C(R³)=N-O-CH₂-, -O-CH₂-C(R³)=N-O-CH₂-, -N=N-C(R3)=N-O-CH₂-, -C(=N-O-R⁵)-C(R³)=N-O-CH₂-, -C(=N-O-R⁵)-C(R³)-O-N=CH-, -C(=N-O-R⁵)-C(R³)-O-N=C(CH₃)-, -T-Ar¹- oder -T-Ar¹-Q- steht,
wobei
n für die Zahlen 0, 1 oder 2 steht,
Q für Sauerstoff oder Schwefel steht,
R³ für Wasserstoff, Cyano, Methyl, Ethyl oder Cyclopropyl steht und
R⁴ für Wasserstoff, Methyl, Ethyl oder Cyclopropyl steht,
R⁵ für Wasserstoff oder Methyl steht,
Ar¹ für jeweils gegebenenfalls einfach bis dreifach, gleich oder ver- schieden substituiertes Phenylen oder Pyridindiyl, für jeweils gegebenenfalls einfach substituiertes Pyrimidindiyl, Pyridazin- diyl, Pyrazindiyl, 1,2,3-Triazindiyl, 1,2,4-Triazindiyl oder 1,3,5- Triazindiyl oder für 1,2,4-Thiadiazoldiyl, 1,3,4-Thiadiazoldiyl, 1,2,4-Oxadiazoldiyl, 1,3,4-Oxadiazoldiyl steht, wobei die mög- lichen Substituenten vorzugsweise aus der nachstehenden Auf- zählung ausgewählt sind:
Fluor, Chlor, Brom, Cyano, Methyl, Ethyl, n- oder i-Propyl, Cyclopropyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluor- ethyl, Difluormethoxy, Trifluormethoxy, Difluorchlormethoxy, Trifluorethoxy, Difluormethylthio, Trifluormethylthio, Difluor- chlormethylthio, Trifluormethylsulfinyl oder Trifluormethyl- sulfonyl und
T für eine Einfachbindung, für Sauerstoff, Schwefel, -CH₂-O-, CH₂-S-, Methylen, Ethylen oder Propylen steht,
Z für Fluor, oder gegebenenfalls einfach bis dreifach, gleich oder verschieden durch Chlor, Cyano oder Methoxy substituiertes Methyl, Ethyl, n- i-Propyl, n-, i-, s- oder t-Butyl;
für Allyl jeweils gegebenenfalls durch Fluor oder Chlor substituiertes Vinyl, oder Propargyl;
für durch stiuiertes oder jeweils gegebenenfalls einfach bis dreifach, gleich oder verschieden Fluor, Chlor, Cyano, Methoxy, Phenyl, Methyl oder Ethyl sub- Cyclopropyl, Cyclobutyl, Cyclopentyl oder Cyclohexyl steht;
Z für jeweils gegebenenfalls einfach bis dreifach, gleich oder verschieden substituiertes Phenyl, 1,2,4-Thiadiazolyl, 1,3,4-Thiadiazolyl, 1,2,4- Oxadiazolyl, 1,3,4-Oxadiazolyl, Pyridinyl, Pyrimidyl, Pyridazinyl, Pyrazinyl, 1,2,3-Triazinyl, 1,2,4-Triazinyl oder 1,3,5-Triazinyl steht, wobei die möglichen Substituenten vorzugsweise aus der nachstehen- den Aufzählung ausgewählt sind:
Fluor, Chlor, Brom, Cyano, Methyl, Ethyl, n- oder i-Propyl, n-, i-, s- oder t-Butyl, Methoxy, Ethoxy, n- oder i-Propoxy, Methylthio, Ethyl- thio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethyl- thio, Trifluormethylthio, Difluorchlormethylthio, Trifluormethylsulfinyl oder Trifluormethylsulfonyl, Methoxycarbonyl, Ethoxycarbonyl,
jeweils gegebenenfalls einfach bis vierfach, gleich oder verschieden durch Fluor, Chlor, Methyl, Trifluormethyl oder Ethyl substituiertes, jeweils zweifach verknüpftes Methylendioxy, Ethylendioxy,
Phenyl, 4-Chlorphenyl, 4-Methylphenyl, Phenoxy, 4-Chlorphenoxy, 4- Methylphenoxy, Cyclopropyl, Cyclobutyl, Cyclopentyl oder Cyclo- hexyl, oder
eine Gruppierung 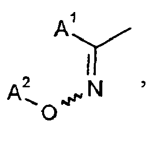 worin
A¹ für Methyl, Ethyl, n- oder i-Propyl, n-, i-, s- oder t-Butyl, Cyclopropyl oder Cyclobutyl steht,
A² für Methyl, Ethyl, n- oder i-Propyl, n-, i-, s- oder t-Butyl, Allyl, Propargyl, But-2-en-1-yl, 2-Methyl-prop-1-en-3-yl, Cyan- methyl, Methoxymethyl, Ethoxymethyl, Methoxyethyl, Eth- oxyethyl, Methylthiomethyl, Ethylthiomethyl, Methylthioethyl, Ethylthioethyl, Dimethylaminomethyl, Dimethylaminoethyl, Methylaminomethyl, Methylaminoethyl oder Benzyl steht, oder eine Gruppierung 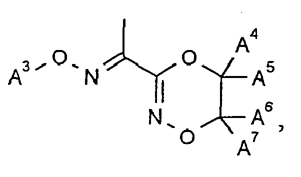 in welcher
A³ für Methyl oder Ethyl steht und
A⁴, A⁵, A⁶ und A⁷ gleich oder verschieden sind und unabhängig voneinander für Wasserstoff, Methyl, Ethyl, n- oder i-Propyl, n- , i-, s- oder t-Butyl, Hydroxymethyl, Trifluormethyl oder Trifluorethyl stehen, oder
A⁴ und A⁵ oder A⁴ und A⁶ oder A⁶ und A⁷ gemeinsam mit den jeweiligen Kohlenstoffatomen, an die sie gebunden sind, einen cycloaliphatischen Ring mit fünf, sechs oder sieben Kohlenstoff- atomen bilden.

Eine besonders bevorzugte Gruppe erfindungsgemäßer Verbindungen sind diejenigen Verbindungen der Formel (I),
in welcher
- R¹: für Wasserstoff, Methyl oder Methoxy steht,
- Y¹, Y², Y³, Y⁴ und Y⁵: gleich oder verschieden sind und für Wasserstoff, Fluor, Chlor, Brom, Cyano, Methyl, Ethyl, Methoxy, Trifluormethyl oder Difluor- methoxy stehen, und entweder
- Y² oder Y³: für -G-Z steht,
worin
G für -C(R³)=N-O-CH₂- steht,
worin
R³ für Methyl oder Cyclopropyl steht, und
Z für jeweils gegebenenfalls einfach bis dreifach, gleich oder verschieden substituiertes Phenyl, Pyridyl oder Pyrimidyl steht, wobei die mögli- chen Substituenten vorzugsweise aus der nachstehenden Aufzählung ausgewählt sind:
Fluor, Chlor, Brom, Cyano, Methyl, Ethyl, n- oder i-Propyl, n-, i-, s- oder t-Butyl, Methoxy, Ethoxy, n- oder i-Propoxy, Methylthio, Ethyl- thio, n- oder i-Propylthio, Methylsulfinyl, Ethylsulfinyl, Methylsulfonyl oder Ethylsulfonyl, Trifluormethyl, Trifluorethyl, Difluormethoxy, Tri- fluormethoxy, Difluorchlormethoxy, Trifluorethoxy, Difluormethylthio, Trifluormethylthio, Difluorchlormethylthio, Trifluormethylsulfinyl oder Trifluormethylsulfonyl, Methoxycarbonyl, Ethoxycarbonyl, Methoximi- nomethyl, Ethoximinomethyl, Methoximinoethyl, Ethoximinoethyl, jeweils gegebenenfalls einfach bis vierfach, gleich oder verschieden durch Fluor, Chlor, Methyl, Trifluormethyl oder Ethyl substituiertes, jeweils zweifach verknüpftes Methylendioxy oder Ethylendioxy, sowie Phenyl und Phenoxy.

Ebenfalls besonders bevorzugte Gruppen erfindungsgemäßer Verbindungen sind diejenigen Verbindungen der Formel (I):
in welcher
- R¹: für Wasserstoff, Methyl oder Methoxy steht.
- Y¹, Y², Y³, Y⁴ und Y⁵: gleich oder verschieden sind und für Wasserstoff, Fluor, Chlor, Brom, Cyano, Methyl, Ethyl, Methoxy, Trifluormethyl oder Difluormethoxy stehen.
- Y² oder Y³: für -G-Z steht,
worin
G für -T-Ar¹- oder -T-Ar¹-O- steht,
worin
Ar¹ für 1,2,4-Thiadiazoldiyl, 1,3,4-Thiadiazoldiyl, 1,2,4-Oxadiazol- diyl, 1,3,4-Oxadiazoldiyl oder für jeweils gegebenenfalls einfach oder zweifach, gleich oder verschieden durch Fluor, Chlor, Cyano, Methyl, Cyclopropyl, Methoxy, Methylthio, Trifluor- methyl, Difluormethoxy, Trifluormethoxy, Difluorchlormethoxy substituiertes, Pyridindiyl, Pyrimidindiyl oder 1,3,5-Triazindiyl steht,
T für eine Einfachbindung, für Sauerstoff, Schwefel, -CH₂-O-, CH₂-S-, Methylen, Ethylen oder Propylen steht.
Z für gegebenenfalls einfach bis dreifach, gleich oder verschieden durch Fluor, Chlor, Cyano oder Methoxy, substituiertes Methyl, Ethyl, n- oder i-Propyl, n-, i-, s- oder t-Butyl;
für jeweils gegebenenfalls durch Fluor oder Chlor substituiertes Vinyl, Allyl oder Propargyl;
für jeweils gegebenenfalls einfach bis dreifach, gleich oder verschieden durch Fluor, Chlor, Cyano, Methoxy, Phenyl, Methyl oder Ethyl sub- stituiertes Cyclopropyl, Cyclobutyl, Cyclopentyl oder Cyclohexyl steht; oder
Z für jeweils gegebenenfalls einfach bis dreifach, gleich oder verschieden substituiertes Phenyl, Pyridyl, Pyrimidyl oder Thienyl steht, wobei die möglichen Substituenten vorzugsweise aus der nachstehenden Auf- zählung ausgewählt sind:
Fluor, Chlor, Brom, Cyano, Methyl, Ethyl, n- oder i-Propyl, n-, i-, s- oder t-Butyl, Methoxy, Ethoxy, n- oder i-Propoxy, Methylthio, Ethyl- thio, n- oder i-Propylthio, Methylsulfinyl, Ethylsulfinyl, Methylsulfonyl oder Ethylsulfonyl, Trifluormethyl, Trifluorethyl, Difluormethoxy, Tri- fluormethoxy, Difluorchlormethoxy, Trifluorethoxy, Difluormethylthio, Trifluormethylthio, Difluorcblonnethylthio, Trifluormethylsulfinyl oder Trifluormethylsulfonyl, Methoxycarbonyl, Ethoxycarbonyl,
jeweils gegebenenfalls einfach bis vierfach, gleich oder verschieden durch Fluor, Chlor, Methyl, Trifluormethyl oder Ethyl substituiertes, jeweils zweifach verknüpftes Methylendioxy, Ethylendioxy
Cyclopropyl, Cyclobutyl, Cyclopentyl, Cyclohexyl, Phenyl, Phenoxy, oder
eine Gruppierung 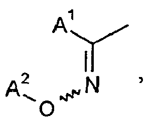 worin
A¹ für Methyl, Ethyl, n- oder i-Propyl, n-, i-, s- oder t-Butyl, Cyclopropyl oder Cyclobutyl steht,
A² für Methyl, Ethyl, n- oder i-Propyl, n-, i-, s- oder t-Butyl, Allyl, Propargyl, But-2-en-1-yl, 2-Methyl-prop-1-en-3-yl, Cyan- methyl, Methoxymethyl, Ethoxymethyl, Methoxyethyl, Eth- oxyethyl, Methylthiomethyl, Ethylthiomethyl, Methylthioethyl, Ethylthioethyl, Dimethylaminomethyl, Dimethylaminoethyl, Methylaminomethyl, Methylaminoethyl oder Benzyl steht, oder
eine Gruppierung 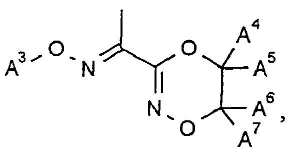 in welcher
A³ für Methyl oder Ethyl steht und
A⁴, A⁵, A⁶ und A⁷ gleich oder verschieden sind und unabhängig voneinander für Wasserstoff, Methyl, Ethyl, n- oder i-Propyl, n-, i-, s- oder t-Butyl, Hydroxymethyl, Trifluormethyl oder Trifluorethyl stehen, oder
A⁴ und A⁵ oder A⁴ und A⁶ oder A⁶ und A⁷ gemeinsam mit den je- weiligen Kohlenstoffatomen, an die sie gebunden sind, einen cycloaliphatischen Ring mit fünf, sechs oder sieben Kohlenstoff- atomen bilden.

Die oben aufgeführten allgemeinen oder in den Vorzugsbereichen angegebenen Restedefinitionen gelten sowohl für die Endprodukte der Formel (I) als auch entsprechend für die jeweils zur Herstellung benötigten Ausgangsstoffe bzw. Zwischenprodukte.

Die in den jeweiligen Kombinationen bzw. bevorzugten Kombinationen von Resten im einzelnen für diese Rest angegebenen Restedefinitionen werden, unabhängig von der jeweilig angegebenen Kombination, beliebig durch entsprechende Restedefinition aus anderen Vorzugsbereichen ersetzt.

Beispielhaft und vorzugsweise werden in den Tabellen 1 bis 4 erfindungsgemäße Verbindungen aufgeführt:

**Tabelle 1**

| | | | | | |
|---|---|---|---|---|---|
| 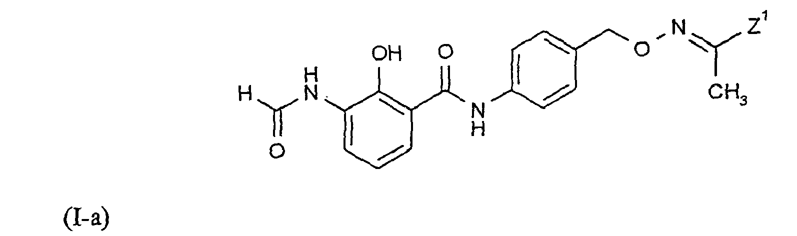 | | | | | |
| | | | | | |
| wobei Z¹ für die folgenden Substituenten steht: | | | | | |
| | | | | | |

| Z¹ | Z¹ | Z¹ | Z¹ | Z¹ | Z¹ |
|---|---|---|---|---|---|
| 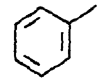 | 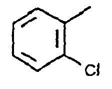 | 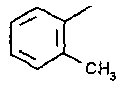 | 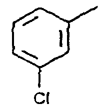 | 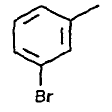 | 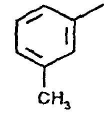 |
| 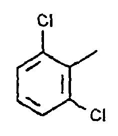 | 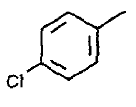 | 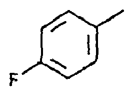 | 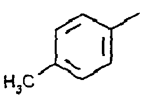 | 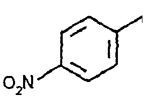 | 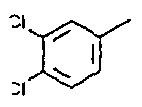 |
| 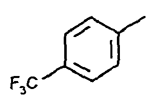 | 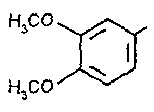 | 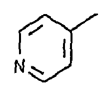 | 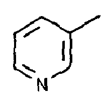 | 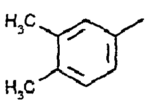 | 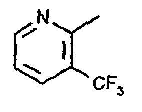 |
| 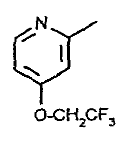 | 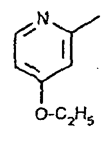 | 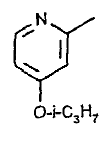 | 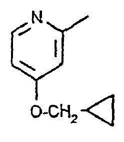 | 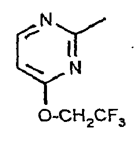 | 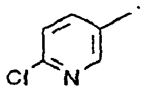 |

**Tabelle 2**

| | | | | | |
|---|---|---|---|---|---|
| 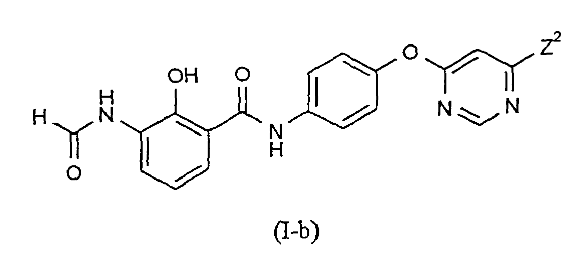 | | | | | |
| | | | | | |
| wobei Z² für die folgenden Substituenten steht: | | | | | |
| | | | | | |

| Z² | Z² | Z² | Z² | Z² | Z² |
|---|---|---|---|---|---|
| 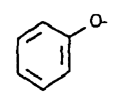 | 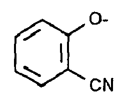 | 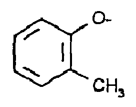 | 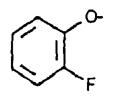 | 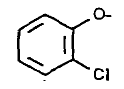 | 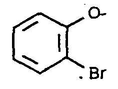 |
| 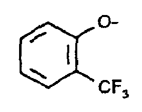 | 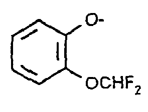 | 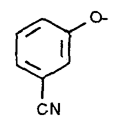 | 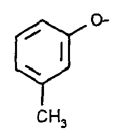 | 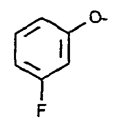 | 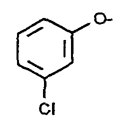 |
| 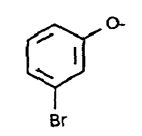 | 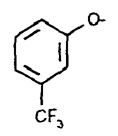 | 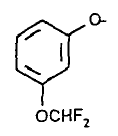 | 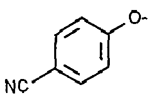 | 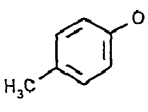 | 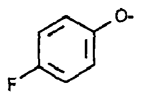 |
| 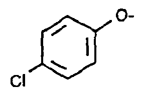 | 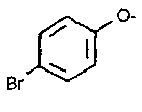 | 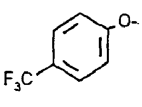 | 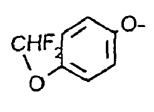 | 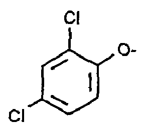 | 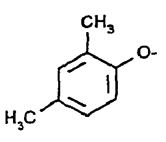 |
| 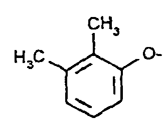 | 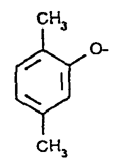 | 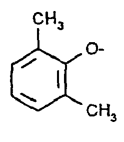 | 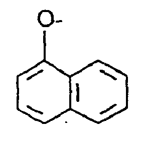 | 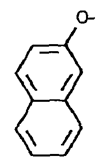 | 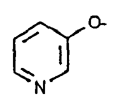 |
| 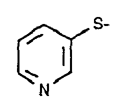 | 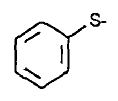 | 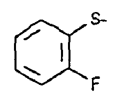 | 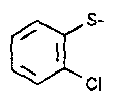 | 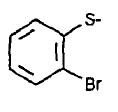 | 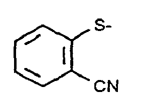 |
| 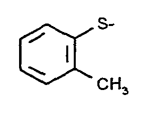 | 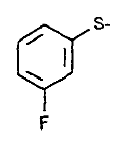 | 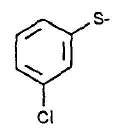 | 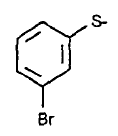 | 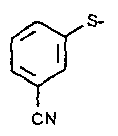 | 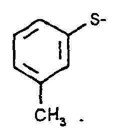 |
| 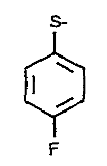 | 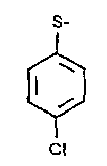 | 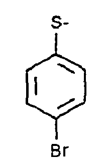 | 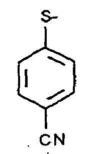 | 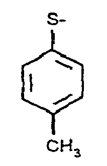 | 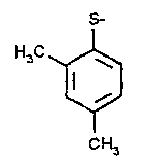 |
| 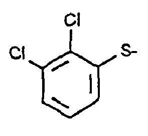 | 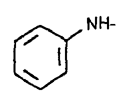 | 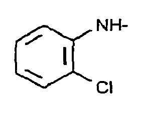 | 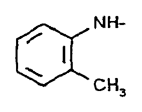 | 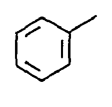 | 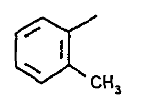 |
| 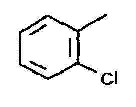 | 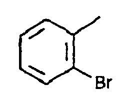 | 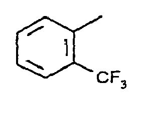 | 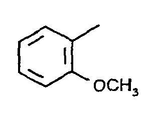 | 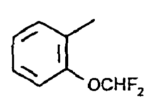 | 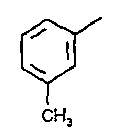 |
| 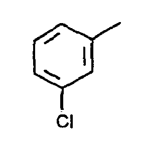 | 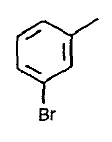 | 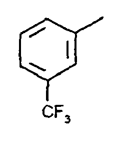 | 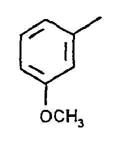 | 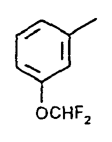 | 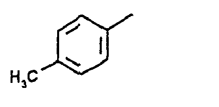 |
| 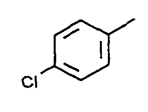 | 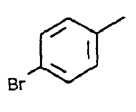 | 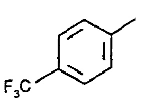 | 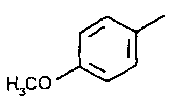 | 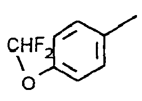 | 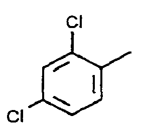 |
| 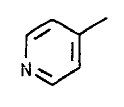 | 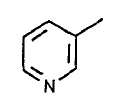 | 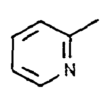 | 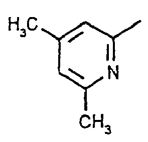 | 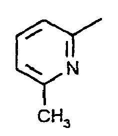 | 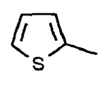 |
| 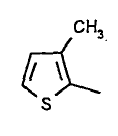 | 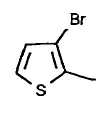 | 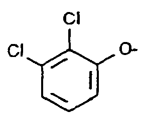 | 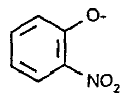 | 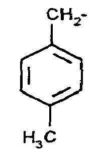 | 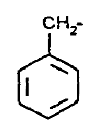 |
| 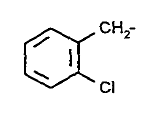 | 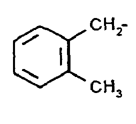 | 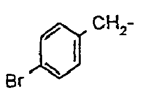 | 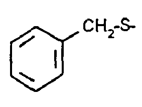 | 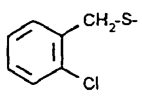 | 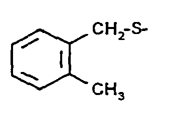 |
| 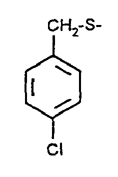 | 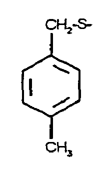 | 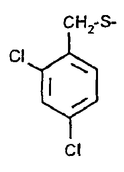 | 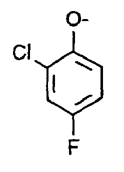 | | |

### Tabelle 3

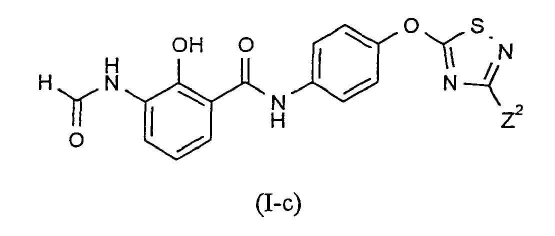

wobei Z² für die in Tabelle 2 genannten Substituenten steht.

### Tabelle 4

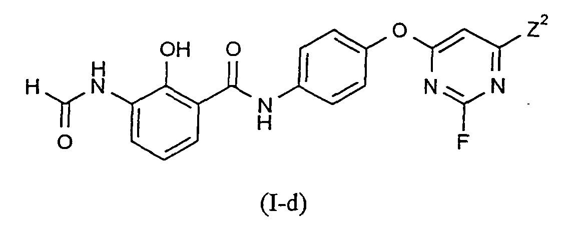

wobei Z² für die in Tabelle 2 genannten Substituenten steht.

Die zur Durchführung des erfindungsgemäßen Verfahrens a) als Ausgangsstoffe benötigten Aminosalicylsäureamide sind durch die Formel (II) allgemein definiert. In dieser Formel (II) haben Y¹, Y², Y³, Y⁴ und Y⁵ vorzugsweise bzw. insbesondere diejenigen Bedeutungen, die bereits im Zusammenhang mit der Beschreibung der erfindungsgemäßen Verbindungen der Formel (I) als bevorzugt bzw. als insbesondere bevorzugt für Y¹, Y², Y³, Y⁴ und Y⁵ angegeben wurden.

Die Ausgangsstoffe der Formel (II) sind neu und ebenfalls Gegenstand der vorliegenden Anmeldung.

Die Aminosalicylsäureamide der Formel (II) werden erhalten, wenn man (Verfahren d) Nitrosalicylsäureamide der allgemeinen Formel (IV), 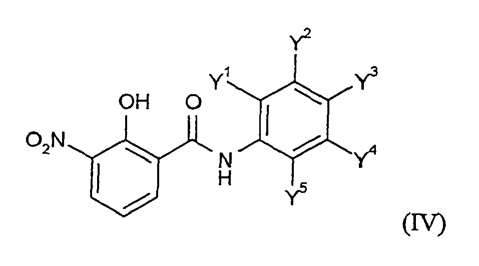 in welcher
Y¹, Y², Y³, Y⁴ und Y⁵ die oben angegebenen Bedeutungen haben,
mit einem Reduktionsmittel, wie beispielsweise Wasserstoff, Eisen, Zink, Zinn-IIchlorid, Natrium- oder Ammoniumhydrogensulfid, gegebenenfalls in Gegenwart eines Verdünnungsmittels und gegebenenfalls in Gegenwart eines Katalysators, umsetzt,
oder wenn man (Verfahren e) O-Benzyl-nitrosalicylsäureamide der allgemeinen Formel (V), 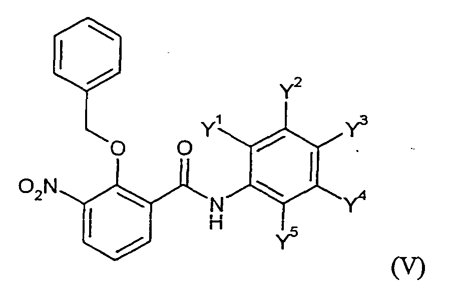 in welcher
Y¹, Y², Y³, Y⁴ und Y⁵ die oben angegebenen Bedeutungen haben,
mit Wasserstoff, gegebenenfalls in Gegenwart eines Verdünnungsmittels, und gegebenenfalls in Gegenwart eines Katalysators, umsetzt.

Die zur Durchführung des erfindungsgemäßen Verfahrens d) als Ausgangsstoffe benötigten Nitrosalicylsäureamide sind durch die Formel (IV) allgemein definiert. In dieser Formel (IV) haben Y¹, Y², Y³, Y⁴ und Y⁵ vorzugsweise bzw. insbesondere diejenigen Bedeutungen, die bereits im Zusammenhang mit der Beschreibung der erfindungsgemäßen Verbindungen der Formel (I) als bevorzugt bzw. als insbesondere bevorzugt für Y¹, Y², Y³, Y⁴ und Y⁵ angegeben wurden.

Die Nitrosalicylsäureamide der Formel (IV) sind neu, und auch Gegenstand der vorliegenden Anmeldung.

Sie werden erhalten, wenn man (Verfahren f) 2-Hydroxy-3-nitrobenzoesäure oder 2-Hydroxy-3-nitrobenzoylchlorid mit einem Amin der Formel (VII), 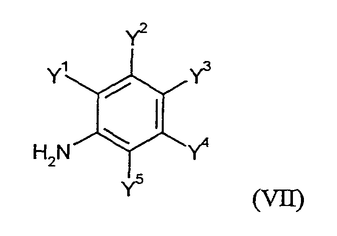 in welcher
Y¹, Y², Y³, Y⁴ und Y⁵ die oben angegebenen Bedeutungen haben,
gegebenenfalls in Gegenwart eines Verdünnungsmittels, gegebenenfalls in Gegenwart eines Kondensationsmittel und gegebenenfalls in Gegenwart eines Säureakzeptors, umsetzt.

Ferner wurde gefunden, daß die neuen Nitrosalicylsäureamide der Formel (IV) sich zur Bekämpfung von Schädlingen an Pflanzen und technischen Materialien, vorzugsweise Pilzen, Insekten und Bakterien, eignen.

Die zur Durchführung des erfindungsgemäßen Verfahrens f) als Ausgangsstoffe benötigte 2-Hydroxy-3-nitrobenzoesäure oder 2-Hydroxy-3-nitrobenzoesäurechlorid sind bekannt (vergleiche z. B. J.Chem.Soc., 1953 2049, 2050 oder US-Patent 03527865).

Die zur Durchführung des erfindungsgemäßen Verfahrens f) weiterhin als Ausgangsstoffe benötigten Amine sind durch die Formel (VII) allgemein definiert. In dieser Formel (VH) haben Y¹, Y², Y³, Y⁴ und Y⁵ vorzugsweise bzw. insbesondere diejenigen Bedeutungen, die bereits im Zusammenhang mit der Beschreibung der erfindungsgemäßen Verbindungen der Formel (I) als bevorzugt bzw. als insbesondere bevorzugt für Y¹, Y², Y³, Y⁴ und Y⁵ angegeben wurden.

Die Amine der Formel (VII) sind teilweise bekannt und können nach bekannten Verfahren hergestellt werden (vergleiche z. B. WO-A 9601825, EP-A 192180).

Neu, und auch Gegenstand der vorliegenden Anmeldung, sind Amine der Formel 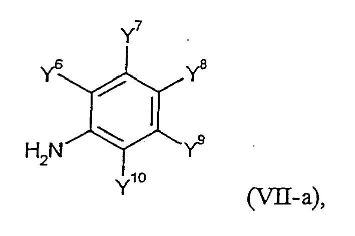 in welcher
- Y⁶, Y⁷, Y⁸, Y⁹ und Y¹⁰: gleich oder verschieden sind und für Wasserstoff, Halogen, Cyano, Alkyl, Halogenalkyl, Alkoxy oder Halogenalkoxy stehen, und ent- weder
- Y⁷ oder Y⁸: für eine Gruppierung 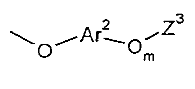 steht,
worin
m für 0 oder 1 steht,
Ar² für 1,2,4-Oxadiazol-3,5-diyl, 1,2,4-Thiadiazol-3,5-diyl oder für gege- benenfalls in 5-Stellung durch Halogen substituiertes Pyrimidin-4,6-diyl steht und
Z³ für gegebenenfalls substituiertes Aryl steht.

Bevorzugt sind Amine der Formel (VII-a),
in welcher
- Y⁶, Y⁷, Y⁸, Y⁹ und Y¹⁰: gleich oder verschieden sind und für Wasserstoff, Halogen, Cyano, Alkyl oder Alkoxy mit jeweils 1 bis 4 Kohlenstoffatomen, Halogen- alkyl, oder Halogenalkoxy mit jeweils 1 bis 4 Kohlenstoffatomen und 1 bis 5 gleichen oder verschiedenen Halogenatomen stehen, und entweder
- Y⁷ oder Y⁸: für eine Gruppierung 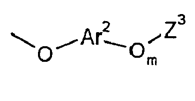 steht,
worin
m für 0 oder 1 steht,
Ar² für 1,2,4-Oxadiazol-3,5-diyl, 1,2,4-Thiadiazol-3,5-diyl oder für gege- benenfalls in 5-Stellung durch Fluor oder Chlor substituiertes Pyrimidin-4,6-diyl steht und
Z³ für jeweils gegebenenfalls einfach oder mehrfach, gleich oder ver- schieden substituiertes Phenyl oder Naphthyl steht, wobei die mög- lichen Substituenten vorzugsweise aus der nachstehenden Aufzählung ausgewählt sind:
Halogen, Cyano, Amino, Hydroxy, Formyl, Carboxy, Carbamoyl, Thiocarbamoyl;
jeweils geradkettiges oder verzweigtes Alkyl, Alkoxy, Alkylthio, Alkyl- sulfinyl oder Alkylsulfonyl mit jeweils 1 bis 6 Kohlenstoffatomen;
jeweils geradkettiges oder verzweigtes Alkenyl oder Alkenyloxy mit jeweils 2 bis 6 Kohlenstoffatomen;
jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschie- denen Halogenatomen;
jeweils geradkettiges oder verzweigtes Halogenalkenyl oder Halogen- alkenyloxy mit jeweils 2 bis 6 Kohlenstoffatomen und 1 bis 11 gleichen oder verschiedenen Halogenatomen;
jeweils geradkettiges oder verzweigtes Alkylamino, Dialkylamino, Alkylcarbonyl, Alkylcarbonyloxy, Alkoxycarbonyl oder Alkylsulfonyl- oxy, mit jeweils 1 bis 6 Kohlenstoffatomen in den einzelnen Alkylteilen;
jeweils gegebenenfalls einfach oder mehrfach, gleich oder verschieden durch Halogen und/oder geradkettiges oder verzweigtes Alkyl mit 1 bis 4 Kohlenstoffatomen und/oder geradkettiges oder verzweigtes Halo- genalkyl mit 1 bis 4 Kohlenstoffatomen und 1 bis 9 gleichen oder ver- schiedenen Halogenatomen substituiertes, jeweils zweifach verknüpftes Alkylen oder Dioxyalkylen mit jeweils 1 bis 6 Kohlenstoffatomen;
Cycloalkyl mit 3 bis 6 Kohlenstoffatomen;
jeweils gegebenenfalls einfach oder mehrfach, gleich oder verschieden durch Halogen und/oder geradkettiges oder verzweigtes Alkyl mit 1 bis 4 Kohlenstoffatomen substituiertes Phenyl oder Phenoxy;
Heterocyclyl oder Heterocyclyl-methyl mit jeweils 3 bis 7 Ringgliedern, von denen jeweils 1 bis 3 gleiche oder verschiedene Heteroatome sind - insbesondere Stickstoff, Sauerstoff und/oder Schwefel - , oder
eine Gruppierung 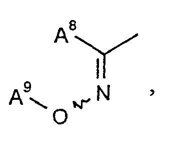 worin
A⁸ für Alkyl mit 1 bis 4 Kohlenstoffatomen oder Cycloalkyl mit 1 bis 6 Kohlenstoffatomen steht und
A⁹ für gegebenenfalls durch Cyano, Alkoxy, Alkylthio, Alkyl- amino, Dialkylamino oder Phenyl substituiertes Alkyl mit 1 bis 4 Kohlenstoffatomen, Alkenyl oder Alkinyl mit jeweils 2 bis 4 Kohlenstoffatomen steht, oder eine Gruppierung 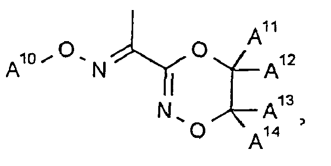 in welcher
A¹⁰ für Alkyl mit 1 bis 4 Kohlenstoffatomen steht und
A¹¹, A¹², A¹³ und A¹⁴ gleich oder verschieden sind und unabhängig voneinander für Wasserstoff, Alkyl oder Hydroxyalkyl mit je- weils 1 bis 4 Kohlenstoffatomen oder Halogenalkyl mit 1 bis 4 Kohlenstoffatomen und 1 bis fünf gleichen oder verschiedenen Halogenatomen stehen, oder
A¹¹ und A¹² oder A¹¹ und A¹³ oder A¹³ und A¹⁴ gemeinsam mit den jeweiligen Kohlenstoffatomen, an die sie gebunden sind, einen cycloaliphatischen Ring mit fünf, sechs oder sieben Kohlenstoffatomen bilden.

Besonders bevorzugt sind Amine der Formel (VII-a),
in welcher
- Y⁶, Y⁷, Y⁸, Y⁹ und Y¹⁰: gleich oder verschieden sind und für Wasserstoff, Fluor, Chlor, Brom, Cyano, Methyl, Ethyl, Methoxy, Trifluormethyl oder Difluormethoxy stehen, und entweder
- Y⁷ oder Y⁸: für eine Gruppierung 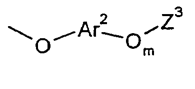 steht,
worin
m für 0 oder 1 steht,
Ar² für 1,2,4-Oxadiazol-3,5-diyl, 1,2,4-Thiadiazol-3,5-diyl oder für gege- benenfalls in 5-Stellung durch Fluor oder Chlor substituiertes Pyri- midin-4, 6-diyl steht und
Z³ für jeweils gegebenenfalls einfach bis dreifach, gleich oder verschieden substituiertes Phenyl steht, wobei die möglichen Substituenten vor- zugsweise aus der nachstehenden Aufzählung ausgewählt sind: Fluor, Chlor, Brom, Cyano, Methyl, Ethyl, n- oder i-Propyl, n-, i-, s- oder t-Butyl, Methoxy, Ethoxy, n- oder i-Propoxy, Methylthio, Ethyl- thio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethyl- thio, Trifluormethylthio, Difluorchlormethylthio, Trifluormethylsulfinyl oder Trifluormethylsulfonyl, Methoxycarbonyl, Ethoxycarbonyl,
jeweils gegebenenfalls einfach bis vierfach, gleich oder verschieden durch Fluor, Chlor, Methyl, Trifluormethyl oder Ethyl substituiertes, jeweils zweifach verknüpftes Methylendioxy, Ethylendioxy, Phenyl, 4-Chlorphenyl, 4-Methylphenyl, Phenoxy, 4-Chlorphenoxy, 4- Methylphenoxy, Cyclopropyl, Cyclobutyl, Cyclopentyl oder Cyclo- hexyl, oder

eine Gruppierung 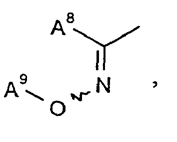 worin
A⁸ für Methyl, Ethyl, n- oder i-Propyl, n-, i-, s- oder t-Butyl, Cyclopropyl oder Cyclobutyl steht,
A⁹ für Methyl, Ethyl, n- oder i-Propyl, n-, i-, s- oder t-Butyl, Allyl, Propargyl, But-2-en-1-yl, 2-Methyl-prop-1-en-3-yl, Cyan- methyl, Methoxymethyl, Ethoxymethyl, Methoxyethyl, Ethoxy- ethyl, Methylthiomethyl, Ethylthiomethyl, Methylthioethyl, Ethylthioethyl, Dimethylaminomethyl, Dimethylaminoethyl, Methylaminomethyl, Methylaminoethyl oder Benzyl steht, oder
eine Gruppierung 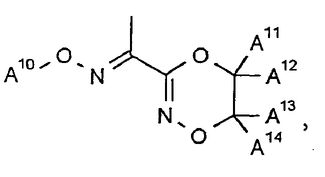 in welcher
A¹⁰ für Methyl oder Ethyl steht und
A¹¹, A¹², A¹³ und A¹⁴ gleich oder verschieden sind und unabhängig voneinander für Wasserstoff, Methyl, Ethyl, n- oder i-Propyl, n-, i-, s- oder t-Butyl, Hydroxymethyl, Trifluormethyl oder Trifluorethyl stehen, oder
A¹¹ und A¹² oder A¹¹ und A¹³ oder A¹³ und A¹⁴ gemeinsam mit den jeweiligen Kohlenstoffatomen, an die sie gebunden sind, einen cycloaliphatischen Ring mit fünf, sechs oder sieben Koh- lenstoffatomen bilden.

Die Amine der Formel (VII-a) werden erhalten (Verfahren h), wenn man Diazole der allgemeinen Formel (VIII) oder Halogenpyrimidine der allgemeinen Formel (IX), 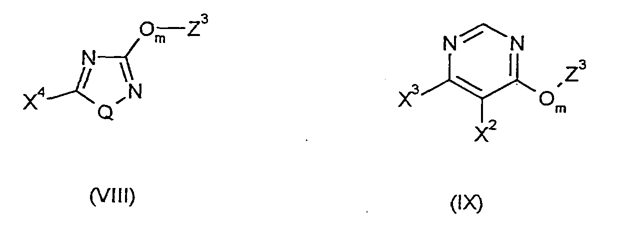 worin
- m: für 0 oder 1 steht,
- X²: für Wasserstoff, Fluor oder Chlor steht,
- X³: für Chlor oder Fluor steht,
- X⁴: für Methylsulfonyl, Chlor oder Brom steht und
- Q: für Sauerstoff oder Schwefel steht, und
- Z³: die oben angegebene Bedeutung hat,
mit einem Aminophenol der Formel, 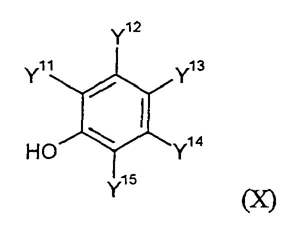 in welcher
- Y¹¹, Y¹², Y¹³, Y¹⁴ und Y¹⁵: gleich oder verschieden sind und für Wasserstoff, Halogen, Cyano, Alkyl, Halogenalkyl, Alkoxy oder Halogenalkoxy stehen, und entweder
- Y¹² oder Y¹³: für Amino steht,
gegebenenfalls in Gegenwart eines Verdünnungsmittels, gegebenenfalls in Gegenwart eines Säureakzeptors und gegebenenfalls in Gegenwart eines Katalysators, umsetzt, oder wenn man (Verfahren i) Aminophenoxypyrimidine der allgemeinen Formel (XI), 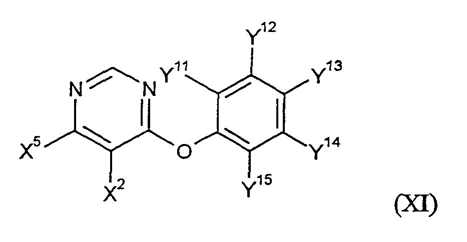 in welcher
- X², Y¹¹, Y¹², Y¹³, Y¹⁴ und Y¹⁵: die oben angegebenen Bedeutungen haben und
- X⁵: für Chlor oder Fluor steht,
mit einem Phenol der allgemeinen Formel (XII),

Z³-OH (XII)

in welcher
- Z³: die oben angegebene Bedeutung hat,
gegebenenfalls in Gegenwart eines Verdünnungsmittels, gegebenenfalls in Gegenwart eines Säureakzeptors und gegebenenfalls in Gegenwart eines Katalysators, umsetzt.

Die zur Durchführung des erfindungsgemäßen Verfahrens h) als Ausgangsstoffe benötigten Diazole sind durch die Formel (VIII), allgemein definiert. In dieser Formel (VIII) haben m und Z³ vorzugsweise bzw. insbesondere diejenigen Bedeutungen, die bereits im Zusammenhang mit der Beschreibung der erfindungsgemäßen Verbindungen der Formel (VII-a) als bevorzugt bzw. als insbesondere bevorzugt für m und Z³ angegeben wurden. X⁴ steht für Methylsulfonyl, Chlor oder Brom.

Die Diazole der Formel (VIII) sind bekannt und können nach bekannten Methoden hergestellt werden (vergleiche z. B. DE-A 2142913).

Die zur Durchführung des erfindungsgemäßen Verfahrens h) als Ausgangsstoffe alternativ benötigten Halogenpyrimidine sind durch die Formel (IX), allgemein definiert. In dieser Formel (IX) haben m und Z³ vorzugsweise bzw. insbesondere diejenigen Bedeutungen, die bereits im Zusammenhang mit der Beschreibung der erfindungsgemäßen Verbindungen der Formel (VII-a) als bevorzugt bzw. als insbesondere bevorzugt für m und Z³ angegeben wurden. X² steht für Wasserstoff, Fluor oder Chlor. X³ steht für Fluor oder Chlor.

Die Halogenpyrimidine der Formel (IX) sind teilweise bekannt und können nach bekannten Methoden hergestellt werden (vgl. z.B. Pharm.Chem.J.(Engl.Transl.), 23, 6, 1989, 500-503; RU, 23, 6, 1989, 705-707).

Neu, und auch Gegenstand der vorliegenden Anmeldung, sind 4-Chlor-5-fluor-6-phenoxypyrimidine der allgemeinen Formel 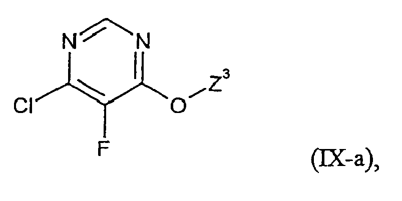 in welcher
- Z³: oben angegebene Bedeutung hat, wobei die Verbindung 2-[(6-Chlor-5-fluor-4- pyrimidinyl)-oxy]-α-(methoxymethylen)-benzoesäuremethylester, ausgenom- men ist.

Die neuen 4-Chlor-5-fluor-6-phenoxypyrimidine werden erhalten, wenn man (Verfahren j) Phenole der allgemeinen Formel (XII),

Z³-OH (XII)

in welcher
- Z³: die oben angegebene Bedeutung hat,
mit 4,6-Dichlor-5-fluorpyrimidin (XIII), gegebenenfalls in Gegenwart eines Verdünnungsmittels, gegebenenfalls in Gegenwart eines Säureakzeptors und gegebenenfalls in Gegenwart eines Katalysators, umsetzt.

Die zur Durchführung des erfindungsgemäßen Verfahrens j) als Ausgangsstoffe benötigten Phenole sind durch die Formel (XII) allgemein definiert. In dieser Formel (XII) hat Z³ vorzugsweise bzw. insbesondere diejenige Bedeutung, die bereits im Zusammenhang mit der Beschreibung der erfindungsgemäßen Verbindungen der Formel (VII-a) als bevorzugt bzw. als insbesondere bevorzugt für Z³ angegeben wurde.

Die Phenole der Formel (XII) sind bekannte Synthesechemikalien oder können nach bekannten Verfahren hergestellt werden (WO 95-04728).

Das zur Durchführung des erfindungsgemäßen Verfahrens j) als Ausgangsstoff weiterhin benötigte 4,6-Dichlor-5-fluorpyrimidin (XIII) ist neu und ebenfalls Gegenstand der vorliegenden Anmeldung.

Es wird erhalten, wenn man (Verfahren k) 5-Fluor-6-hydroxy-4(1H)-pyrimidinon (XTV) mit einem Chlorierungsmittel, gegebenenfalls in Gegenwart eines Verdünnungsmittels und gegebenenfalls in Gegenwart eines Katalysators, umsetzt.

Das zur Durchführung des erfindungsgemäßen Verfahrens k) als Ausgangsstoff benötigte 5-Fluor-6-hydroxy-4(1H)-pyrimidinon (XIV) ist bekannt und kann nach bekannten Methoden hergestellt werden (JP 61205262; CA: 106:84632).

Die zur Durchführung des erfindungsgemäßen Verfahrens i) als Ausgangsstoffe benötigten Aminophenoxypyrimidine sind durch die Formel (XI) allgemein definiert. In dieser Formel (XI) haben Y¹¹, Y¹², Y¹³, Y¹⁴ und Y¹⁵ vorzugsweise bzw. insbesondere diejenigen Bedeutungen, die bereits im Zusammenhang mit der Beschreibung der Amine der Formel (X) als bevorzugt bzw. als insbesondere bevorzugt für Y¹¹, Y¹², Y¹³, Y¹⁴ und Y¹⁵ angegeben wurden. X² und X⁵ stehen unabhängig voneinander für Chlor oder Fluor.

Die Aminophenoxypyrimidine der Formel (XI) sind neu und ebenfalls Gegenstand der vorliegenden Anmeldung.

Sie werden erhalten, wenn man (Verfahren 1) Trihalogenpyrimidine der Formel (XV), 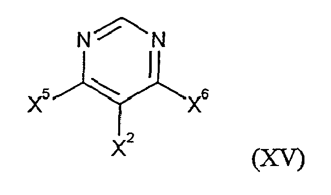 in welcher
- X² und X⁵: die oben angegebenen Bedeutungen haben und
- X⁶: für Fluor oder Chlor steht,
mit einem Aminophenol der Formel (X),
gegebenenfalls in Gegenwart eines Verdünnungsmittels, gegebenenfalls in Gegenwart eines Säureakzeptors und gegebenenfalls in Gegenwart eines Katalysators, umsetzt.

Die zur Durchführung des erfindungsgemäßen Verfahrens 1) als Ausgangsstoffe benötigten Trihalogenpyrimidine sind durch die Formel (XV) allgemein definiert. In dieser Formel (XV) haben X² und X⁵ vorzugsweise bzw. insbesondere diejenigen Bedeutungen, die bereits im Zusammenhang mit der Beschreibung der Verbindungen der Formel (XI) als bevorzugt bzw. als insbesondere bevorzugt für X² und X⁵ angegeben wurden. X⁶ steht für Fluor oder Chlor.

Die Trihalogenpyrimidine der Formel (XV) sind teilweise bekannt und können nach bekannten Methoden hergestellt werden (vergleiche z. B. Chesterfield et al., J. Chem. Soc., 1955; 3478, 3480). Ein Sonderfall der Verbindungen der Formel (XV) ist die erfindungsgemäße Verbindung (XIII); sie kann nach Verfahren k) hergestellt werden.

Die zur Durchführung des erfindungsgemäßen Verfahrens 1) weiterhin als Ausgangsstoffe benötigten Aminophenole der Formel (X) sind bereits weiter oben bei der Beschreibung des erfindungsgemäßen Verfahrens h) beschrieben worden.

Die zur Durchführung des erfindungsgemäßen Verfahrens i) weiterhin als Ausgangsstoffe benötigten Phenole der Formel (XII) sind bereits weiter oben bei der Beschreibung des erfindungsgemäßen Verfahrens j) beschrieben worden.

Die zur Durchführung des erfindungsgemäßen Verfahrens e) als Ausgangsstoffe benötigten O-Benzyl-nitrosalicylsäureamide sind durch die Formel (V), allgemein definiert. In dieser Formel (V) haben Y¹, Y², Y³, Y⁴ und Y⁵ vorzugsweise bzw insbesondere diejenigen Bedeutungen, die bereits im Zusammenhang mit der Beschreibung der erfindungsgemäßen Verbindungen der Formel (I) als bevorzugt bzw. als insbesondere bevorzugt für Y¹, Y², Y³, Y⁴ und Y⁵ angegeben wurden.

Die O-Benzyl-nitrosalicylsäureamide der Formel (V) sind noch nicht bekannt, sie sind als neue Stoffe Gegenstand der vorliegenden Anmeldung.

Die O-Benzyl-nitrösalicylsäureamide der Formel (V) werden erhalten, wenn man (Verfahren g) O-Benzyl-nitrosalicylsäurederivate der Formel (VI), 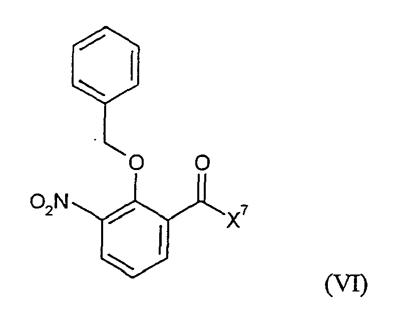 in welcher
- X⁷: für Halogen, Hydroxy oder Alkoxy steht,
mit einem Amin der Formel (VII),
gegebenenfalls in Gegenwart eines Verdünnungsmittels, gegebenenfalls in Gegenwart eines Kondensationsmittel und gegebenenfalls in Gegenwart eines Säureakzeptors, umsetzt.

Die zur Durchführung des erfindungsgemäßen Verfahrens g) als Ausgangsstoffe benötigten O-Benzyl-nitrosalicylsäurederivate sind durch die Formel (VI), allgemein definiert. In dieser Formel (VI) steht X⁷ für Halogen, vorzugsweise Chlor; Hydroxy oder Alkoxy, vorzugsweise Methoxy oder Ethoxy.

Die O-Benzyl-nitrosalicylsäurederivate der Formel (VI) sind bekannt und können nach bekannten Methoden hergestellt werden (vergleiche z. B. J. Am Chem. Soc. 1959, 5215-5217).

Die zur Durchführung des erfindungsgemäßen Verfahrens g) weiterhin als Ausgangsstoffe benötigten Amine der Formel (VII) bereits weiter oben im Zusammenhang mit der Beschreibung des erfindungsgemäßen Verfahrens f) beschrieben worden.

Die zur Durchführung des erfindungsgemäßen Verfahrens a) weiterhin als Ausgangsstoffe benötigten Acylierungsmittel sind durch die Formel (III), allgemein definiert. In dieser Formel (III) hat R¹ vorzugsweise bzw. insbesondere diejenige Bedeutung, die bereits im Zusammenhang mit der Beschreibung der erfindungsgemäßen Verbindungen der Formel (I) als bevorzugt bzw. als insbesondere bevorzugt für R¹ angegeben wurde. X¹ steht für Halogen, Hydroxy, Alkoxy oder Alkylcarbonyloxy, vorzugsweise für Chlor, Hydroxy, Methoxy, Ethoxy oder Acetoxy.

Die Acylierungsmittel der allgemeinen Formel (III) sind bekannte Reagenzien in der organischen Chemie.

Die zur Durchführung des erfindungsgemäßen Verfahrens b) als Ausgangsstoffe benötigten Nitrosalicylsäureamide der Formel (IV) sind bereits weiter oben im Zusammenhang mit der Beschreibung des erfindungsgemäßen Verfahrens d) beschrieben worden.

Die zur Durchführung des erfindungsgemäßen Verfahrens c) als Ausgangsstoffe benötigten O-Benayl-nitrosalicylsäureamide der Formel (V) sind bereits weiter oben im Zusammenhang mit der Beschreibung des erfindungsgemäßen Verfahrens e) beschrieben worden.

Als Verdünnungsmittel zur Durchführung der erfindungsgemäßen Verfahren a), f) und g) kommen alle inerten organischen Lösungsmittel in Betracht. Hierzu gehören vorzugsweise aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methyl-t-Amylether, Dioxan, Tetrahydrofuran, 1,2- Dimethoxyethan, 1,2-Diethoxyethan oder Anisol; Ketone, wie Aceton, Butanon, Methyl-isobutylketon oder Cyclohexanon; Nitrile, wie Acetonitril, Propionitril, n- oder i-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid; Ester wie Essigsäuremethylester oder Essigsäureethylester; Sulfoxide, wie Dimethylsulfoxid oder Sulfone, wie Sulfolan.

Als Verdünnungsmittel zur Durchführung der erfindungsgemäßen Verfahren d)und e) kommen alle inerten organischen Lösungsmittel in Betracht. Hierzu gehören vorzugsweise Ester wie Essigsäuremethylester oder Essigsäureethylester; Alkohole, wie Methanol, Ethanol, n- oder i-Propanol, n-, i-, sek- oder tert-Butanol, Ethandiol, Propan-1,2-diol, Ethoxyethanol, Methoxyethanol, Diethylenglykolmonomethylether, Diethylenglykolmonoethylether, Wasser, Salzlösungen, wie beispielsweise Ammoniumchloridlösung, Säuren, wie beispielsweise Salzsäure oder Essigsäure, sowie beliebige Mischungen der genannten Verdünnungsmittel.

Als Verdünnungsmittel zur Durchführung der erfindungsgemäßen Verfahren h), i), j) und 1) kommen alle inerten organischen Lösungsmittel in Betracht. Hierzu gehören vorzugsweise Ether, wie Diethylether, Diisopropylether, Methyl-t-butylether, Methyl-t-Amylether, Dioxan, Tetrahydrofuran, 1,2- Dimethoxyethan, 1,2-Diethoxyethan oder Anisol; Nitrile, wie Acetonitril, Propionitril, n- oder i-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid; Sulfoxide, wie Dimethylsulfoxid; oder Sulfone, wie Sulfolan.

Als Verdünnungsmittel zur Durchführung des erfindungsgemäßen Verfahrens k) kommen alle inerten organischen Lösungsmittel in Betracht. Hierzu gehören vorzugsweise aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Dichlormethan, Chloroform, Tetrachlormethan, Dichlorethan oder Trichlorethan; Nitrile, wie Acetonitril, Propionitril, n- oder i-Butyronitril oder Benzonitril.

Die erfindungsgemäßen Verfahren a), f) und g) werden gegebenenfalls in Gegenwart eines geeigneten Säureakzeptors durchgeführt. Als solche kommen alle üblichen anorganischen oder organischen Basen infrage. Hierzu gehören vorzugsweise Erdalkalimetall- oder Alkalimetallhydroxide, -acetate, -carbonate oder -hydrogencarbonate, wie beispielsweise Natriumhydroxid, Kaliumhydroxid, Natriumacetat, Kaliumacetat, Calciumacetat, Natriumcarbonat, Kaliumcarbonat, Kaliumhydrogencarbonat oder Natriumhydrogencarbonat sowie tertiäre Amine, wie Trimethylamin, Triethylamin, Tributylamin, N,N-Dimethylaailin, N,N-Dimethyl-benzylamin, Pyridin, N-Methylpiperidin, N-Methylmorpholin, N,N-Dimethylammopyridin, Diazabicyclooctan (DABCO), Diazabicyclononen (DBN) oder Diazabicycloundecen (DBU).

Die erfindungsgemäßen Verfahren b), c), d) und e) werden gegebenenfalls in Gegenwart eines Katalysators durchgeführt. Als solche kommen alle Katalysatoren infrage, die auch für Hydrierungen üblicherweise verwendet werden. Beispielhaft seien genannt: Raney-Nickel, Palladium oder Platin, gegebenenfalls auf einem Trägermaterial, wie beispielsweise Aktivkohle.

Das erfindungsgemäße Verfahren c) wird gegebenenfalls auch in Gegenwart von Wasserstoff oder gegebenenfalls in Gegenwart eines unedlen Metalls durchgeführt. Als unedle Metalle seien beispielhaft genannt: Zink, Zinn, Eisen, Aluminium oder Magnesium.

Als weitere Reaktionshilfsmittel zur Durchführung der erfindungsgemäßen Verfahren a), b) und c) kommen alle wasserentziehenden Mittel, insbesondere Essigsäureanhydrid, infrage.

Die erfindungsgemäßen Verfahren f) und g) werden gegebenenfalls in Gegenwart eines Kondensationsmittels durchgeführt. Hierzu gehören vorzugsweise Säurehalogenidbildner wie beispielsweise Phosgen, Phosphortribromid, Phosphortrichlorid, Phosphorpentachlorid, Phosphoroxychlorid oder Thionylchlorid; Anhydridbildner wie Chlorameisensäureethylester, Chlorameisensäuremethylester, Chlorameisensäureisopropylester, Chlorameisensäureisobutylester oder Methansulfonylchlorid; Carbodiimide, wie N,N'-Dicyclohexylcarbodiimid (DCC) oder andere übliche Kondensationsmittel, wie Phosphorpentoxid, Polyphosphorsäure, N,N'-Carbonyldiimidazol, 2-Ethoxy-N-ethoxycarbonyl-1,2-dihydrochinolin (EEDQ) oder Triphenylphosphin/Tetrachlormethan.

Die erfindungsgemäßen Verfahren h), i), j) und 1) werden gegebenenfalls in Gegenwart eines geeigneten Säureakzeptors durchgeführt. Als solche kommen alle üblichen anorganischen oder organischen Basen infrage. Hierzu gehören vorzugsweise Erdalkalimetall- oder Alkalimetallhydride, -hydroxide, -alkoholate, -carbonate oder -hydrogencarbonate, wie beispielsweise Natriumhydrid, Natriumamid, Kalium-tert.-butylat, Natriumhydroxid, Kaliumhydroxid, Natriumcarbonat, Kaliumcarbonat, Kaliumhydrogencarbonat oder Natriumhydrogencarbonat.

Als Katalysatoren für die erfindungsgemäßen Verfahren h), i), j) und 1) eignen sich alle Kupfer(1)-Salze, wie beispielsweise Kupfer(I)-chlorid, Kupfer(I)-bromid oder Kupfer-(I)-iodid, oder auch Fluoride, wie beispielsweise Natrium-, Kalium- oder Ammoniumfluorid, sowie tertiäre Ammoniumfluoride, wie Tetrabutylammoniumfluorid.

Das erfindungsgemäße Verfahren k) wird gegebenenfalls in Gegenwart eines geeigneten Katalysators durchgeführt. Als solche kommen alle tertiäre Amine infrage, wie beispielsweise Trimethylamin, Triethylamin, Tributylamin, N,N-Dimethylanilin, N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU), sowie Amide, wie Dimethylformamid.

Als Halogenierungsmittel zur Durchführung des erfindungsgemäßen Verfahrens k) kommen alle Reagenzien infrage, die an Kohlenstoff gebundene Hydroxygruppen gegen Chlor austauschen können. Beispielhaft seien genannt: Phosgen, Phosphortrichlorid, Phosphorpentachlorid, Phosphoroxychlorid oder Thionylchlorid und gegebenenfalls zusätzlich Chlorwasserstoff oder Chlor.

Die Reaktionstemperaturen können bei der Durchführung der erfindungsgemäßen Verfahren a), b), c), d), e), f) und g) in einem größeren Bereich variiert werden. Im allgemeinen arbeitet man bei Temperaturen von 0°C bis 180°C, vorzugsweise bei Temperaturen von 0°C bis 130°C.

Die Reaktionstemperaturen können bei der Durchführung der erfindungsgemäßen Verfahren h), i), j) und I) in einem größeren Bereich variiert werden. Im allgemeinen arbeitet man bei Temperaturen von -20°C bis 200°C, vorzugsweise bei Temperaturen von -10°C bis 150°C.

Die Reaktionstemperaturen können bei der Durchführung des erfindungsgemäßen Verfahrens k) in einem größeren Bereich variiert werden. Im allgemeinen arbeitet man bei Temperaturen von 0°C bis 250°C, vorzugsweise bei Temperaturen von 0°C bis 200°C.

Zur Durchführung des erfindungsgemäßen Verfahrens a) zur Herstellung der Verbindungen der Formel (I) setzt man pro Mol des Aminosalicylsäureamids der Formel (II) im allgemeinen 1 bis 2000 Mol, vorzugsweise 1 bis 800 Mol Acylierungsmittel der Formel (III) ein.

Zur Durchführung der erfindungsgemäßen Verfahren b) und c) zur Herstellung der Verbindungen der Formel (I) setzt man pro Mol des Nitrosalicylsäureamides der Formel (IV), bzw. des O-Benzyl-nitrosalicylsäureamides der Formel (V) im allgemeinen 100 bis 2000 Mol, vorzugsweise 200 bis 1000 Mol Ameisensäure ein.

Zur Durchführung des erfindungsgemäßen Verfahrens d) zur Herstellung der Verbindungen der Formel (II) setzt man pro Mol des Nitrosalicylsäureamides der Formel (IV) im allgemeinen 1 bis 100 Mol, vorzugsweise 2 bis 20 Mol Reduktionsmittel ein.

Zur Durchführung des erfindungsgemäßen Verfahrens e) zur Herstellung der Verbindungen der Formel (II) setzt man pro Mol des O-Benzyl-nitrosalicylsäureamides der Formel (V) im allgemeinen 1 bis 100 Mol, vorzugsweise 2 bis 50 Mol Wasserstoff ein. Zur Durchführung des erfindungsgemäßen Verfahrens f) zur Herstellung der Verbindungen der Formel (IV) setzt man pro Mol Amin der Formel (VII) im allgemeinen 0,5 bis 10 Mol, vorzugsweise 0,8 bis 5 Mol 2-Hydroxy-3-nitrobenzoesäure oder 2-Hydroxy-3-nitrobenzoylchlorid ein.

Zur Durchführung des erfindungsgemäßen Verfahrens g) zur Herstellung der Verbindungen der Formel (V) setzt man pro Mol des Amins der Formel (VII) im allgemeinen 0,5 bis 10 Mol, vorzugsweise 0,8 bis 5 Mol O-Benzyl-nitrosalicylsäurederivate der Formel (VI) ein.

Zur Durchführung des erfindungsgemäßen Verfahrens h) zur Herstellung der Verbindungen der Formel (VII-a) setzt man pro Mol des Aminophenols der Formel (X) im allgemeinen 0,5 bis 5 Mol, vorzugsweise 0,8 bis 2 Mol Halogenpyrimidin der Formel (EX), bzw. Diazol der Formel (VIII) ein.

Zur Durchführung des erfindungsgemäßen Verfahrens i) zur Herstellung der Verbindungen der Formel (VII-a) setzt man pro Mol des Phenols der Formel (XII) im allgemeinen 0,5 bis 5 Mol, vorzugsweise 0,8 bis 2 Mol Aminophenoxypyrimidin (XI) ein.

Zur Durchführung des erfindungsgemäßen Verfahrens j) zur Herstellung der Verbindungen der Formel (IX-a) setzt man pro Mol des Phenols der Formel (XII) im allgemeinen 0,5 bis 5 Mol, vorzugsweise 0,8 bis 2 Mol 4,6-Dichlor-5-fluorpyrimidin (XIII) ein.

Zur Durchführung des erfindungsgemäßen Verfahrens k) zur Herstellung von 4,6-Dichlor-5-fluorpyrimidin (XIII) setzt man pro Mol 5-Fluor-6-hydroxy-4(1H)-pyrimidinon (XIV) im allgemeinen 0,05 bis 20 Mol, vorzugsweise 0,1 bis 10 Mol Halogenierungsmittel ein.

Zur Durchführung des erfindungsgemäßen Verfahrens 1) zur Herstellung der Verbindungen der Formel (XI) setzt man pro Mol des Aminophenols der Formel (X) im allgemeinen 0,5 bis 5 Mol, vorzugsweise 0,8 bis 2 Mol Trihalogenpyrimidin (XV) ein.

Die erfindungsgemäßen Verfahren a) bis 1) werden im allgemeinen unter Normaldruck durchgeführt. Es ist jedoch auch möglich, unter erhöhtem oder vermindertem Druck - im allgemeinen zwischen 0,1 bar und 10 bar - zu arbeiten.

Die erfindungsgemäßen Stoffe weisen eine starke mikrobizide Wirkung auf und können zur Bekämpfung von unerwünschten Mikroorganismen, wie Fungi und Bakterien, im Pflanzenschutz und im Materialschutz eingesetzt werden.

Fungizide lassen sich Pflanzenschutz zur Bekämpfung von Plasmodiophoromycetes, Oomycetes, Chytridiomycetes, Zygomycetes, Ascomycetes, Basidiomycetes und Deuteromycetes einsetzen.

Bakterizide lassen sich im Pflanzenschutz zur Bekämpfung von Pseudomonadaceae, Rhizobiaceae, Enterobacteriaceae, Corynebacteriaceae und Streptomycetaceae einsetzen.

Beispielhaft aber nicht begrenzend seien einige Erreger von pilzlichen und bakteriellen Erkrankungen, die unter die oben aufgezählten Oberbegriffe fallen, genannt:
Xanthomonas-Arten, wie beispielsweise Xanthomonas campestris pv. oryzae;
Pseudomonas-Arten, wie beispielsweise Pseudomonas syringae pv. lachrymans;
Erwinia-Arten, wie beispielsweise Erwinia amylovora;
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Bremia-Arten, wie beispielsweise Bremia lactucae;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea (Konidienform: Drechslera, Syn: Helminthosporium);
Cochliobolus-Arten, wie beispielsweise Cochliobolus sativus (Konidienform Drechslera, Syn: Helminthosporium);
Uromyces-Arten, wie beispielsweise Uromyces appendiculatus;
Puccinia-Arten, wie beispielsweise Puccinia recondita;
Sclerotinia-Arten, wie beispielsweise Sclerotinia sclerotiorum;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Gercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei lassen sich die erfindungsgemäßen Wirkstoffe mit besonders gutem Erfolg zur Bekämpfung von Krankheiten im Wein-, Obst- und Gemüseanbau, wie beispielsweise gegen Venturia- und Phytophtora-Arten, einsetzen. Mit gutem Erfolg werden auch Getreidekrankheiten, wie beispielsweise, Pseudocercosporella-Arten oder Reiskrankheiten, wie beispielsweise Pyricularia-Arten, bekämpft.

Die erfindungsgemäßen Wirkstoffe eignen sich auch zur Steigerung des Ernteertrages. Sie sind außerdem mindertoxisch und weisen eine gute Pflanzenverträglichkeit auf

Die Wirkstoffe können in Abhängigkeit von ihren jeweiligen physikalischen und/oder chemischen Eigenschaften in die üblichen Formulierungen überführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycolether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die Wirkstoffe können in Abhängigkeit von ihren jeweiligen physikalischen und/oder chemischen Eigenschaften in die üblichen Formulierungen überführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Warmnebel-Formulierungen.

Die erfindungsgemäßen Wirkstoffe können als solche oder in ihren Formulierungen auch in Mischung mit bekannten Fungiziden, Bakteriziden, Akariziden, Nematiziden oder Insektiziden verwendet werden, um so z.B. das Wirkungsspektrum zu verbreitern oder Resistenzentwicklungen vorzubeugen. In vielen Fällen erhält man dabei synergistische Effekte, d.h. die Wirksamkeit der Mischung ist größer als die Wirksamkeit der Einzelkomponenten.

Als Mischpartner kommen zum Beispiel folgende Verbindungen in Frage:
Fungizide:
   Aldimorph, Ampropylfos, Ampropylfos-Kalium, Andoprim, Anilazin, Azaconazol, Azoxystrobin,
   Benalaxyl, Benodanil, Benomyl, Benzamacril, Benzamacryl-isobutyl, Bialaphos, Binapacryl, Biphenyl, Bitertanol, Blasticidin-S, Bromuconazol, Bupirimat, Buthiobat,
   Calciumpolysulfid, Capsimycin, Captafol, Captan, Carbendazim, Carboxin, Carvon, Chinomethionat (Quinomethionat), Chlobenthiazon, Chlorfenazol, Chloroneb, Chloropicrin, Chlorothalonil, Chlozolinat, Clozylacon, Cufraneb, Cymoxanil, Cyproconazol, Cyprodinil, Cyprofuram,
   Debacarb, Dichlorophen, Diclobutrazol, Diclofluanid, Diclomezin, Dicloran, Diethofencarb, Difenoconazol, Dimethirimol, Dimethomorph, Diniconazol, Diniconazol-M, Dinocap, Diphenylamin, Dipyrithione, Ditalimfos, Dithianon, Dodemorph, Dodine, Drazoxolon,
   Ediphenphos, Epoxiconazol, Etaconazol, Ethirimol, Etridiazol,
   Famoxadon, Fenapanil, Fenarimol, Fenbuconazol, Fenfuram, Fenitropan, Fenpiclonil, Fenpropidin, Fenpropimorph, Fentinacetat, Fentinhydroxyd, Ferbam, Ferimzon, Fluazinam, Flumetover, Fluoromid, Fluquinconazol, Flurprimidol, Flusilazol, Flusulfamid, Flutolanil, Flutriafol, Folpet, Fosetyl-Alminium, Fosetyl-Natrium, Fthalid, Fuberidazol, Furalaxyl, Furametpyr, Furcarbonil, Furconazol, Furconazol-cis, Furmecyclox,
   Guazatin,
   Hexachlorobenzol, Hexaconazol, Hymexazol,
   Imazalil, Imibenconazol, Iminoctadin, Iminoctadinealbesilat, Iminoctadinetriacetat, Iodocarb, Ipconazol, Iprobenfos (IBP), Iprodione, Irumamycin, Isoprothiolan, Isovaledione,
   Kasugamycin, Kresoxim-methyl, Kupfer-Zubereitungen, wie: Kupferhydroxid, Kupfernaphthenat, Kupferoxychlorid, Kupfersulfat, Kupferoxid, Oxin-Kupfer und Bordeaux-Mischung,
   Mancopper, Mancozeb, Maneb, Meferimzone, Mepanipyrim, Mepronil, Metalaxyl, Metconazol, Methasulfocarb, Methfuroxam, Metiram, Metomeclam, Metsulfovax, Mildiomycin, Myclobutanil, Myclozolin,
   Nickel-dimethyldithiocarbamat, Nitrothal-isopropyl, Nuarimol,
   Ofurace, Oxadixyl, Oxamocarb, Oxolinicacid, Oxycarboxim, Oxyfenthiin,
   Paclobutrazol, Pefurazoat, Penconazol, Pencycuron, Phosdiphen, Pimaricin, Piperalin, Polyoxin, Polyoxorim, Probenazol, Prochloraz, Procymidon, Propamocarb, Propanosine-Natrium, Propiconazol, Propineb, Pyrazophos, Pyrifenox, Pyrimethanil, Pyroquilon, Pyroxyfur,
   Quinconazol, Quintozen (PCNB),
   Schwefel und Schwefel-Zubereitungen,
   Tebuconazol, Tecloftalam, Tecnazen, Tetcyclacis, Tetraconazol, Thiabendazol, Thicyofen, Thifluzamide, Thiophanate-methyl, Thiram, Tioxymid, Tolclofos-methyl, Tolylfluanid, Triadimefon, Triadimenol, Triazbutil, Triazoxid, Trichlamid, Tricyclazol, Tridemorph, Triflumizol, Triforin, Triticonazol,
   Uniconazol,
   Validamycin A, Vinclozolin, Viniconazol,
   Zarilamid, Zineb, Ziram sowie
   Dagger G,
   OK-8705,
   OK-8801,
   α-(1,1-Dimethylethyl)-β-(2-phenoxyethyl)-1H-1,2,4-triazol-1-ethanol,
   α-(2,4-Dichlorphenyl)-β-fluor-b-propyl-1H-1,2,4-triazol-1-ethanol,
   α-(2,4-Dichlorphenyl)-β-methoxy-a-methyl-1H-1,2,4-triazol-1-ethanol,
   α-(5-Methyl-1,3-dioxan-5-yl)-ß-[[4-(trifluormethyl)-phenyl]-methylen]-1H-1,2,4-triazol-1-ethanol,
   (5RS,6RS)-6-Hydroxy-2,2,7,7-tetramethyl-5-(1H-1,2,4-triazol-1-yl)-3-octanon,
   (E)-a-(Methoxyimino)-N-methyl-2-phenoxy-phenylacetamid,
   {2-Methyl-1-[[[1-(4-methylphenyl)-ethyl]-amino]-carbonyl]-propyl}-carbaminsäure-1-isopropylester
   1-(2,4-Dichlorphenyl)-2-(1H-1,2,4-triazol-1-yl)-ethanon-O-(phenylmethyl)-oxim,
   1-(2-Methyl-1-naphthalenyl)-1H-pyrrol-2,5-dion,
   1-(3,5-Dichlorphenyl)-3-(2-propenyl)-2,5-pyrrolidindion,
   1-[(Diiodmethyl)-sulfonyl]-4-methyl-benzol,
   1-[[2-(2,4-Dichlorphenyl)-1,3-dioxolan-2-yl]-methyl]-1H-imidazol,
   1-[[2-(4-Chlorphenyl)-3-phenyloxiranyl]-methyl]-1H-1,2,4-triazol,
   1-[1-[2-[(2,4-Dichlorphenyl)-methoxy]-phenyl]-ethenyl]-1H-imidazol,
   1-Methyl-5-nonyl-2-(phenylmethyl)-3-pyrrolidinol,
   2',6'-Dibrom-2-methyl-4'-trifluormethoxy-4'-trifluor-methyl-1,3-thiazol-5-carboxanilid,
   2,2-Dichlor-N-[1-(4-chlorphenyl)-ethyl]-1-ethyl-3-methyl-cyclopropancarboxamid,
   2,6-Dichlor-5-(methylthio)-4-pyrimidinyl-thiocyanat,
   2,6-Dichlor-N-(4-trifluormethylbenzyl)-benzamid,
   2,6-Dichlor-N-[[4-(trifluormethyl)-phenyl]-methyl]-benzamid,
   2-(2,3,3-Triiod-2-propenyl)-2H-tetrazol,
   2-[(1-Methylethyl)-sulfonyl]-5-(trichlormethyl)-1,3,4-thiadiazol,
   2-[[6-Deoxy-4-O-(4-O-methyl-ß-D-glycopyranosyl)-a-D-glucopyranosyl]-amino]-4-methoxy-1H-pyrrolo[2,3-d]pyrimidin-5-carbonitril,
   2-Aminobutan,
   2-Brom-2-(brommethyl)-pentandinitril,
   2-Chlor-N-(2,3-dihydro-1,1,3-trimethyl-1H-inden-4-yl)-3-pyridincarboxamid,
   2-Chlor-N-(2,6-dimethylphenyl)-N-(isothiocyanatomethyl)-acetamid,
   2-Phenylphenol(OPP),
   3,4-Dichlor-1-[4-(difluormethoxy)-phenyl]-1H-pyrrol-2,5-dion,
   3,5-Dichlor-N-[cyan[(1-methyl-2-propynyl)-oxy]-methyl]-benzamid,
   3-(1,1-Dimethylpropyl-1-oxo-1H-inden-2-carbonitril,
   3-[2-(4-Chlorphenyl)-5-ethoxy-3-isoxazolidinyl]-pyridin,
   4-Chlor-2-cyan-N,N-dimethyl-5-(4-methylphenyl)-1H-imidazol-1-sulfonamid,
   4-Methyl-tetrazolo[1,5-a]quinazolin-5(4H)-on,
   8-(1,1-Dimethylethyl)-N-ethyl-N-propyl-1,4-dioxaspiro[4.5]decan-2-methanamin,
   8-Hydroxychinolinsulfat,
   9H-Xanthen-9-carbonsäure-2-[(phenylamino)-carbönyl]-hydrazid,
   bis-(1-Methylethyl)-3-methyl-4-[(3-methylbenzoyl)-oxy]-2,5-thiophendicarboxylat,
   cis-1-(4-Chlorphenyl)-2-(1H-1,2,4-triazol-1-yl)-cycloheptanol,
   cis-4-[3-[4-(1,1-Dimethylpropyl)-phenyl-2-methylpropyl]-2,6-dimethyl-morpholin-hydrochlorid,
   Ethyl-[(4-chlorphenyl)-azo]-cyanoacetat,
   Kaliumhydrogencarbonat,
   Methantetrathiol-Natriumsalz,
   Methyl-1-(2,3-dihydro-2,2-dimethyl-1H-inden-1-yl)-1H-imidazol-5-carboxylat,
   Methyl-N-(2,6-dimethylphenyl)-N-(5-isoxazolylcarbonyl)-DL-alaninat,
   Methyl-N-(chloracetyl)-N-(2,6-dimethylphenyl)-DL-alaninat,
   N-(2,3-Dichlor-4-hydroxyphenyl)-1-methyl-cyclohexancarboxamid.
   N-(2,6-Dimethylphenyl)-2-methoxy-N-(tetrahydro-2-oxo-3-furanyl)-acetamid,
   N-(2,6-Dimethylphenyl)-2-methoxy-N-(tetrahydro-2-oxo-3-thienyl)-acetamid,
   N-(2-Chlor-4-nitrophenyl)-4-methyl-3-nitro-benzolsulfonamid,
   N-(4-Cyclohexylphenyl)-1,4,5,6-tetrahydro-2-pyrimidinamin,
   N-(4-Hexylphenyl)-1,4,5,6-tetrahydro-2-pyrimidinamin,
   N-(5-Chlor-2-methylphenyl)-2-methoxy-N-(2-oxo-3-oxazolidinyl)-acetamid,
   N-(6-Methoxy)-3-pyridinyl)-cyclopropancarboxamid,
   N-[2,2,2-Trichlor-1-[(chloracetyl)-amino]-ethyl]-benzamid,
   N-[3-Chlor-4,5-bis-(2-propinyloxy)-phenyl]-N-methoxy-methanimidamid,
   N-Formyl-N-hydroxy-DL-alanin -Natriumsalz,
   O,O-Diethyl-[2-(dipropylamino)-2-oxoethyl]-ethylphosphoramidothioat,
   O-Methyl-S-phenyl-phenylpropylphosphoramidothioate,
   S-Methyl-1,2,3-benzothiadiazol-7-carbothioat,
   spiro[2H]-1-Benzopyran-2,1'(3'H)-isobenzofuran]-3'-on,
**Bakterizide:**
   Bronopol, Dichlorophen, Nitrapyrin, Nickel-dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.
**Insektizide / Akarizide / Nematizide:**
   Abamectin, Acephat, Acrinathrin, Alanycarb, Aldicarb, Alphamethrin, Amitraz, Avermectin, AZ 60541, Azadirachtin, Azinphos A, Azinphos M, Azocyclotin,
   Bacillus thuringiensis, 4-Bromo-2-(4-chlorphenyl)-1-(ethoxymethyl)-5-(trifluoromethyl)-1H-pyrrole-3-carbonitrile, Bendiocarb, Benfuracarb, Bensultap, Betacyfluthrin, Bifenthrin, BPMC, Brofenprox, Bromophos A, Bufencarb, Buprofezin, Butocarboxim, Butylpyridaben,
   Cadusafos, Carbaryl, Carbofuran, Carbophenothion, Carbosulfan, Cartap, Chloethocarb, Chlorethoxyfos, Chlorfenapyr, Chlorfenvinphos, Chlorfluazuron, Chlormephos, N-[(6-Chloro-3-pyridinyl)-methyl]-N-cyano-N-methyl-ethanimidamide, Chlorpyrifos, Chlorpyrifos M, Cis-Resmethrin, Clocythrin, Clofentezin, Cyanophos, Cycloprothrin, Cyfluthrin, Cyhalothrin, Cyhexatin, Cypermethrin, Cyromazin,
   Deltamethrin, Demeton M, Demeton S, Demeton-S-methyl, Diafenthiuron, Diazinon, Dichlofenthion, Dichlorvos, Dicliphos, Dicrotophos, Diethion, Diflubenzuron, Dimethoat, Dimethylvinphos, Dioxathion, Disulfoton,
   Edifenphos, Emamectin, Esfenvalerat, Ethiofencarb, Ethion, Ethofenprox, Ethoprophos, Etrimphos,
   Fenamiphos, Fenazaquin, Fenbutatinoxid, Fenitrothion, Fenobucarb, Fenothiocarb, Fenoxycarb, Fenpropathrin, Fenpyrad, Fenpyroximat, Fenthion, Fenvalerate, Fipronil, Fluazinam, Fluazuron, Flucycloxuron, Flucythrinat, Flufenoxuron, Flufenprox, Fluvalinate, Fonophos, Formothion, Fosthiazat, Fubfenprox, Furathiocarb,
   HCH, Heptenophos, Hexaflumuron, Hexythiazox,
   Imidacloprid, Iprobenfos, Isazophos, Isofenphos, Isoprocarb, Isoxathion, Ivermectin,
   Lamda-cyhalothrin, Lufenuron,
   Malathion, Mecarbam, Mevinphos, Mesulfenphos, Metaldehyd, Methacrifos, Methamidophos, Methidathion, Methiocarb, Methomyl, Metolcarb, Milbemectin, Monocrotophos, Moxidectin,
   Naled, NC 184, Nitenpyram
   Omethoat, Oxamyl, Oxydemethon M, Oxydeprofos,
   Parathion A, Parathion M, Permethrin, Phenthoat, Phorat, Phosalon, Phosmet, Phosphamidon, Phoxim, Pirimicarb, Pirimiphos M, Pirimiphos A, Profenophos, Promecarb, Propaphos, Propoxur, Prothiophos, Prothoat, Pymetrozin, Pyrachlophos, Pyridaphenthion, Pyresmethrin, Pyrethrum, Pyridaben, Pyrimidifen, Pyriproxifen,
   Quinalphos,
   Salithion, Sebufos, Silafluofen, Sulfotep, Sulprofos,
   Tebufenozide, Tebufenpyrad, Tebupirimiphos, Teflubenzuron, Tefluthrin, Temephos, Terbam, Terbufos, Tetrachlorvinphos, Thiafenox, Thiodicarb, Thiofanox, Thiomethon, Thionazin, Thuringiensin, Tralomethrin, Triarathen, Triazophos, Triazuron, Trichlorfon, Triflumuron, Trimethacarb,
   Vamidothion, XMC, Xylylcarb, Zetamethrin.
   Auch eine Mischung mit anderen bekannten Wirkstoffen, wie Herbiziden oder mit Düngemitteln und Wachstumsregulatoren ist möglich.

Die Wirkstoffe können als solche, in Form ihrer Formulierungen oder den daraus bereiteten Anwendungsformen, wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Beim Einsatz der erfindungsgemäßen Wirkstoffe als Fungizide können die Aufwandmengen je nach Applikationsart innerhalb eines größeren Bereiches variiert werden. Bei der Behandlung von Pflanzenteilen liegen die Aufwandmengen an Wirkstoff im allgemeinen zwischen 0,1 und 10.000 g/ha, vorzugsweise zwischen 10 und 1.000 g/ha. Bei der Saatgutbehandlung liegen die Aufwandmengen an Wirkstoff im allgemeinen zwischen 0,001 und 50 g pro Kilogramm Saatgut, vorzugsweise zwischen 0,01 und 10 g pro Kilogramm Saatgut. Bei der Behandlung des Bodens liegen die Aufwandmengen an Wirkstoff im allgemeinen zwischen 0,1 und 10.000 g/ha, vorzugsweise zwischen 1 und 5.000 g/ha.

### Herstellungsbeispiele:

### Beispiel (1)

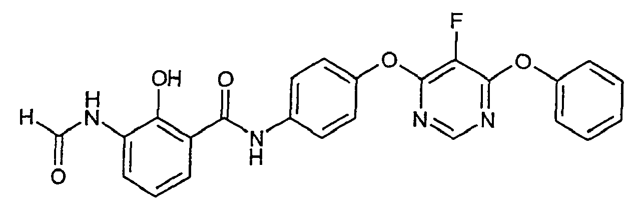

### Verfahren c)

1,67 g (3,02 mMol) 2-Benzyloxy-N-[4-(5-fluoro-6-phenoxy-pyrimidin-4-yloxy)-phenyl]-3-nitro-benzamid werden in 20 ml Ameisensäure mit 0,4 g Raney-Nickel 24 Stunden bei 40°C gerührt. Die Mischung wird auf Wasser gegeben und mehrfach mit Essigsäureethylester extrahiert. Die vereinigten organischen Phasen werden über Natriumsulfat getrocknet und bei vermindertem Druck eingeengt. Der Rückstand wird mit Cyclohexan/Essigester (1:1) an Kieselgel chromatografiert. Man erhält 0,4 g (35 % der Theorie) N-[4-(5-Fluor-6-phenoxy-pyrimidin-4-yloxy)-phenyl]-3-formyl-amino-2-hydroxy-benzamid als farbloses Öl.
HPLC: logP = 3,31

### Beispiel (1)

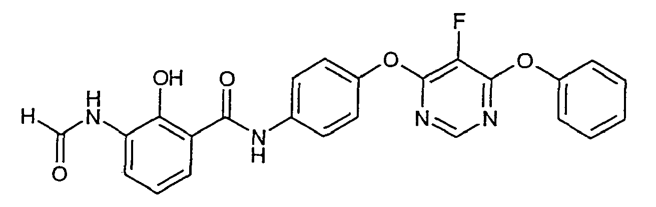

### Verfahren b)

0,5 g (1,08 mmol) N-[4-(5-Fluor-6-phenoxy-pyrimidin-4-yloxy)-phenyl]-2-hydroxy-3-nitrobenzamid werden in 10 ml Ameisensäure mit 0,5 g Raney-Nckel 24 Stunden bei 40°C gerührt. Die Mischung wird auf Wasser gegeben und mehrfach mit Essigsäureethylester extrahiert. Die vereinigten organischen Phasen werden über Natriumsulfat getrocknet und bei vermindertem Druck eingeengt. Der Rückstand wird mit Cyclohexan/Essigester (1:1) an Kieselgel chromatografiert. Man erhält 0,4 g (81 % der Theorie) N-[4-(5-Fluor-6-phenoxy-pyrimidin-4-yloxy)-phenyl]-3-formyl-amino-2-hydroxy-benzamid als farbloses Öl.
HPLC: logP = 3,31

### Herstellung von Verbindungen der Formel (IV)

### Beispiel (IV-1):

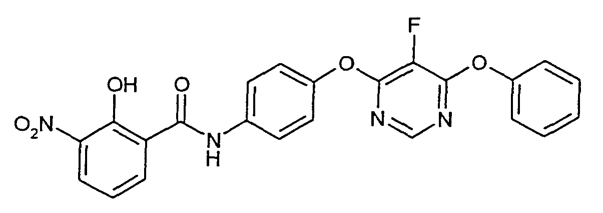

### Verfahren f)

Eine Mischung aus 1,7 g (9 mMol) 3-Nitrosalicylsäure und 2,7 g (9 mMol) 4-(5-Fluor-6-phenoxy-pyrimidin-4-yloxy)-phenylamin in 20 ml Toluol wird unter Rühren auf 50°C erhitzt. Hierzu gibt man 0,5 g Phosphortrichlorid und erhitzt weitere 4 Stunden unter Rückfluß zum Sieden. Nach dem Abkühlen wird das Gemisch eingeengt und der Rückstand mit Dichlormethan extrahiert. Die organische Phase wird mehrfach mit Wasser gewaschen und über Natriumsulfat getrocknet. Nach dem Abdestillieren des Lösungsmittels erhält man 1,7 g (41% der Theorie ) N-[4-(5-Fluor-6-phenoxy-pyrimidin-4-yloxy)-phenyl]-2-hydroxy-3-nitrobenzamid.
Massenspektrum: m/e = 462 (M⁺)

### Herstellung von Verbindungen der Formel (V)

### Beispiel (V-1):

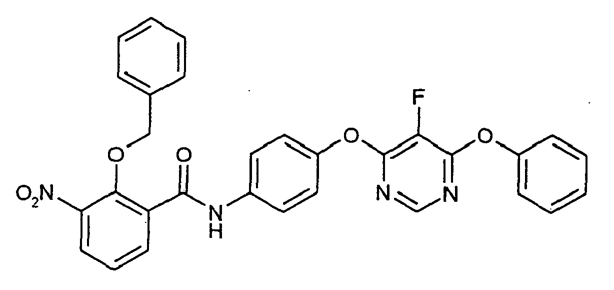

### Verfahren g)

Zu einer Lösung von 4,3 g (18 mMol) 2-Benzyloxy-3-nitro-benzoylchlorid in 20 ml Dichlormethan tropft man bei 0°C eine Lösung von 2,4 g (0,011 Mol) Triethylamin und 5,3 g (18 mMol) 4-(5-Fluor-6-phenoxy-pyrimidin-4-yloxy)-phenylamin in 30 ml Dichlormethan und rührt noch 2 Stunden ohne weitere Kühlung. Die Mischung wird filtriert, das Filtrat mit Wasser gewaschen, über Natriumsulfat getrocknet und bei vermindertem Druck eingeengt. Der Rückstand wird mit Cyclohexan/Essigester (2: 1) an Kieselgel chromatografiert. Man erhält 2,8 g (30% der Theorie) 2-Benzyloxy-N-[4-(5-fluoro-6-phenoxy-pyrimidin-4-yloxy)-phenyl]-3-nitro-benzamid als Öl.
HPLC: logP = 4,56

### Herstellung von Verbindungen der Formel (IX-a)

### Beispiel (IX-a-1):

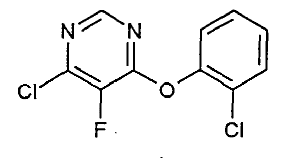

### Verfahren j)

Zu einer Mischung von 5 g (0,0295 Mol) 4,6-Dichlor-5-fluorpyrimidin und 5 g (0,036 Mol) Kaliumcarbonat in 25 ml Acetonitril wird auf 50 °C erwärmt. Bei dieser Temperatur tropft man innerhalb von 6 Stunden eine Lösung von 3,8 g (0,03 Mol) 2-Chlorphenol in 25 ml Acetonitril und rührt weitere 6 Stunden bei dieser Temperatur. Nach dem Abkühlen wird das Lösungsmittel bei vermindertem Druck abdestilliert und der Rückstand mit Wasser aufgenommen und mehrmals mit jeweils 40 ml Dichlormethan extrahiert. Die organische Phase wird mit 10%iger Natronlauge gewaschen, über Natriumsulfat getrocknet und wiederum bei vermindertem Druck eingeengt. Man erhält 6,9 g (86,7% der Theorie) 4-Chlor-6-(2-chlorphenoxy)-5-fluorpyrimidin.
¹H-NMR: δ = 7,24-7,53 (m, 4H); 8,29 (s, 1H) ppm

### Beispiel (IX-a-2):

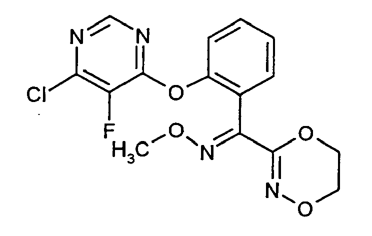

### Verfahren j)

Zu einer Mischung von 3 g (0,018 Mol) 4,6-Dichlor-5-fluorpyrimidin und 2,7 g (0,018 Mol) Kaliumcarbonat in 30 ml Acetonitril wird auf 70°C erwärmt. Bei dieser Temperatur tropft man innerhalb von 2 Stunden eine Lösung von 3,8 g (0,03 Mol) (5,6-Dihydro-[1,4,2]dioxazin-3-yl)-(2-hydroxy-phenyl)-methanon-O-methyl-oxim in 20 ml Acetonitril und erhitzt weitere 16 Stunden unter Rückfluß. Nach dem Abkühlen wird das Lösungsmittel bei vermindertem Druck abdestilliert und der Rückstand mit Wasser aufgenommen und mehrmals mit jeweils 40 ml Dichlormethan extrahiert. Die organische Phase wird mit 10%iger Natronlauge gewaschen, über Natriumsulfat getrocknet und wiederum bei vermindertem Druck eingeengt. Man erhält 7 g (86,7% der Theorie) [2-(6-Chlor-5-fluorpyrimidin-4-yloxy)-phenyl]-(5,6-dihydro-[1,4,2]-dioxazin-3-yl)-methanon-O-methyloxim.
¹H-NMR: δ = 3,83 (s, 3H); 4,14 (m, 2H); 4,45 (m, 2H); 7,39-7,54 (m, 4H); 8,32 (s, 1H) ppm

### Herstellung von 4,6-Dichlor-5-fluorpyrimidin

### Beispiel (XIII):

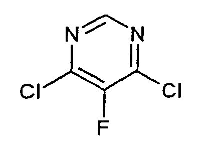

### Verfahren k)

Zu 30 ml Phosphoroxychlorid wird innerhalb von 30 Minuten 7,8 g (0,064 Mol) N,N-Dimethylanilin getropft und 10 Minuten bei 25 °C gerührt. Anschließend gibt man 8,3 g 5-Fluorpyrimidin-4,6-diol zu und erhitzt 15 Stunden unter Rückfluß. Nach dem Abkühlen wird das überschüssige Phosphoroxychlorid im Wasserstrahlvakuum abdestilliert und der Rückstand einer Vakuumdestillation unterworfen. Man erhält 11,2 g (100 % der Theorie) 4,6-Dichlor-5-fluorpyrimidin vom Siedepunkt 58 - 60°C bei 14 mbar.

### Herstellung von Verbindungen der Formel (VII-a)

### Beispiel (VII-a-1):

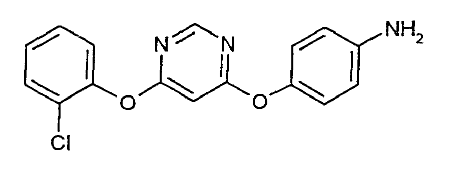

### Verfahren i)

Eine Mischung aus 2 g (9 mMol) 4-(4-Aminophenoxy)-6-chlor-pyrimidin, 1,1 g (9 mMol) 3-Chlorphenol und 2 g (18 mMol) getrocknetes Kaliumcarbonat in 20 ml Acetonitril wird 4 Stunden unter Rückfluß erhitzt. Anschließend wird die Mischung filtriert und das Filtrat bei vermindertem Druck eingeengt. Der Rückstand wird in Dichlormethan aufgenommen, mit Wasser gewaschen, die organische Phase über Natriumsulfat getrocknet und wiederum bei vermindertem Druck eingeengt. Man erhält 1,3 g (46 % der Theorie) 4-(4-Aminophenoxy)-6-(2-chlorphenoxy)-pyrimidin.
HPLC: logP = 1,96

### Herstellung von Verbindungen der Formel (XI)

### Beispiel (XI-1):

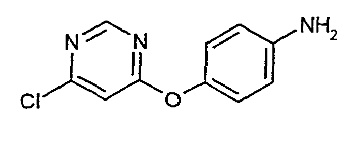

### Verfahren 1)

Eine Mischung aus 50 g (0,3 Mol) 4,6-Dichlorpyrimidin, 37 g (0,3 Mol) 4-Aminophenol, 80 g (0,6 Mol) getrocknetes Kaliumcarbonat und 50 mg Kupfer-II-bromid in 600 ml Acetonitril wird 4 Stunden unter Rückfluß erhitzt. Anschließend wird die Mischung filtriert und das Filtrat bei vermindertem Druck eingeengt. Der Rückstand wird in Dichlormethan aufgenommen, mit Wasser gewaschen, die organische Phase über Natriumsulfat getrocknet und wiederum bei vermindertem Druck eingeengt. Man erhält 60 g (58 % der Theorie) 4-(4-Aminophenoxy)-6-chlor-pyrimidin.
HPLC: logP = 0,65

### Beispiel (VII-a-2):

### Verfahren h)

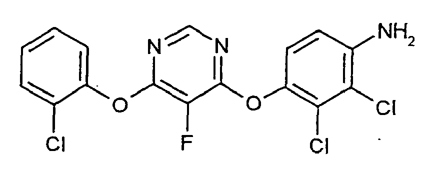

2,7 g 6-Chlor-5-fluor-4-(2-chlorphenyloxy)pyrimidin und 1,5 g Kaliumcarbonat werden in 20 ml Acetonitril vorgelegt und unter Rückfluß innerhalb von 1,5 Stunden mit 1,8 g 2,3-Dichlor-4-aminophenol versetzt. Man erhitzt weitere 12 Stunden unter Rückfluß, kühlt ab, entfernt das Lösungsmittel im Vakuum und nimmt den Rückstand in 45 ml Dichlormethan auf Nach dem Waschen mit 0.25 N wässriger Natronlauge versetzt man die Lösung mit 5 g Aktivkohle, filtriert und destilliert das Lösungsmittel ab. Der Rückstand wird an Kieselgel mit Petrolether/Essigester (2:1) chromatographiert.
Man erhält 2,7 g (64,3 %) an 2,3-Dichlor-4-[6-(2-chlorphenoxy)-5-fluorpyrimidin-4-yloxy]-phenylamin vom Schmelzpunkt 162-163°C.

Analog Beispiel 1, sowie entsprechend der allgemeinen Beschreibung der erfindungsgemäßen Verfahren b) und c) wurden die in der nachfolgenden Tabelle 5 genannten Verbindungen der Formel (I-f) erhalten:

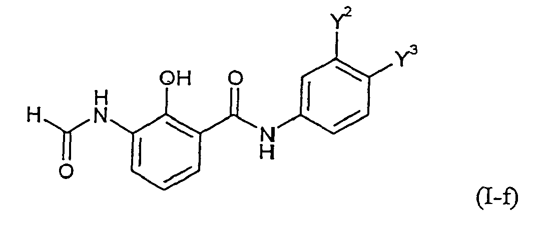

**Tabelle 5**

| Beispiel | Y² | Y³ | logP |
|---|---|---|---|
| 2 | -H | 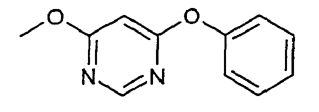 | 2,96 |
| 3 | -H | 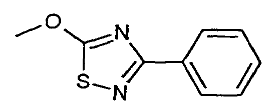 | 3,62 |
| 4 | -H | 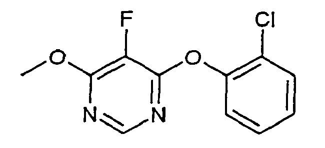 | 3,61 |
| 5 | -CH₃ | 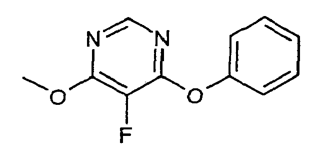 | 3,35 |
| 6 | -H | 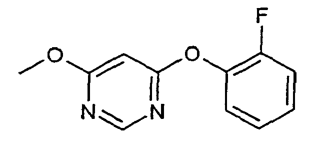 | 3,07 |
| 7 | -H | 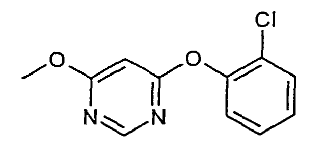 | 3,26 |
| 8 | -H | 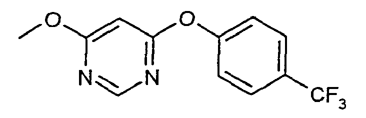 | 3,21 |
| 9 | -H | 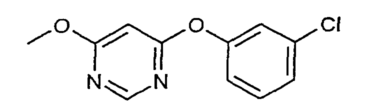 | 3,86 |
| 10 | -H | 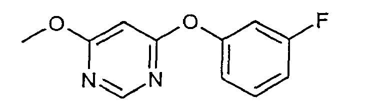 | 3,09 |
| 11 | -H | 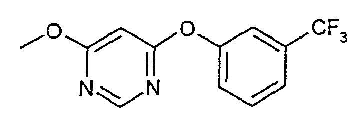 | 3,58 |
| 12 | -H | 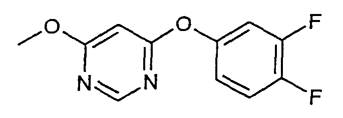 | 3,19 |
| 13 | -H | 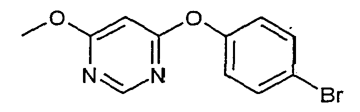 | 3,53 |
| 14 | -H | 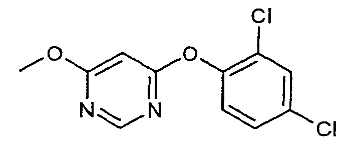 | 3,86 |
| 15 | -H | 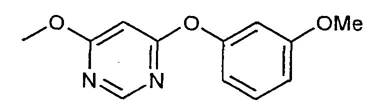 | 2,95 |
| 16 | -H | 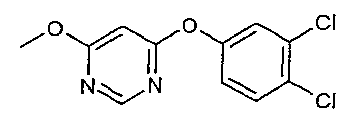 | 3,86 |
| 17 | -H | 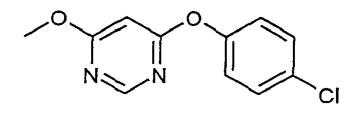 | 3,39 |
| 18 | -H | 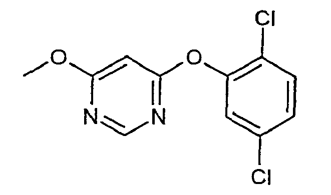 | 3,79 |
| 19 | -H | 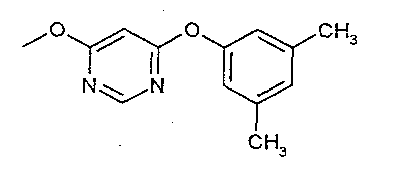 | 3,61 |
| 20 | -H | 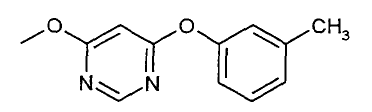 | 3,27 |
| 21 | -H | 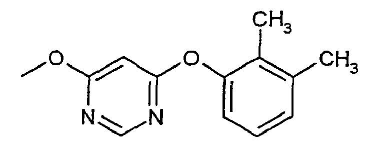 | 3,59 |
| 22 | -H | 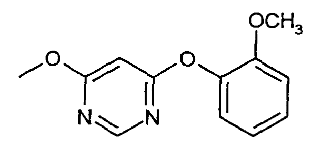 | 2,82 |
| 23 | -H | 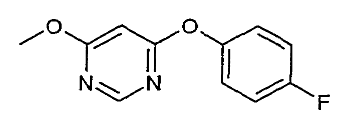 | 3,00 |
| 24 | -H | 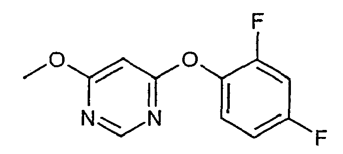 | 3,17 |
| 25 | -H | 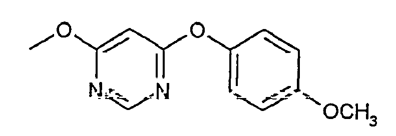 | 2,88 |
| 26 | -H | 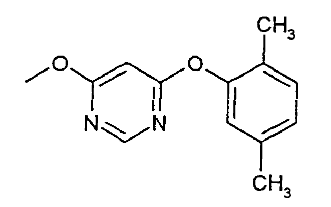 | 3,55 |
| 27 | -H | 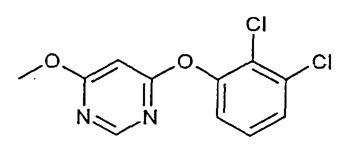 | 4,09 |
| 28 | -H | 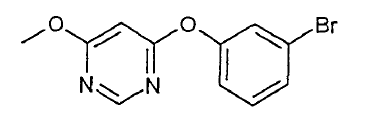 | 3,58 |
| 29 | -H | 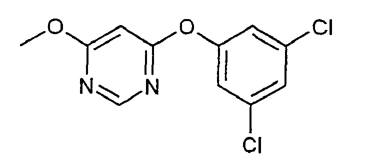 | 4,10 |
| 30 | -H | 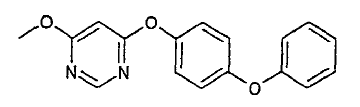 | 3,88 |
| 31 | -H | 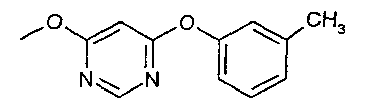 | 3,20 |
| 32 | -H | 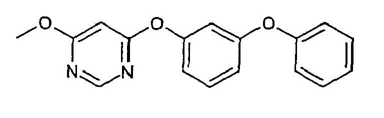 | 3,90 |

Analog Beispiel (IV-1), sowie entsprechend der allgemeinen Beschreibung des erfindungsgemäßen Verfahrens f) wurden die in der nachfolgenden Tabelle 6 genannten Verbindungen der Formel (IV-a) erhalten:

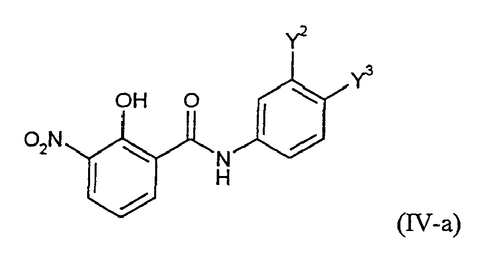

**Tabelle 6**

| Beispiel | Y² | Y³ | phys. Daten |
|---|---|---|---|
| (IV-2) | -H | 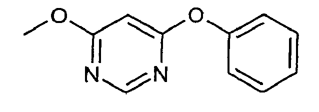 | MS*: m/e = 444 (M⁺) |

| | | | |
|---|---|---|---|
| * Massenspektrum | | | |

Analog Beispiel (V-1), sowie entsprechend der allgemeinen Beschreibung des erfindungsgemäßen Verfahrens g) wurden die in der nachfolgenden Tabelle 7 genannten Verbindungen der Formel (VI-a) erhalten:

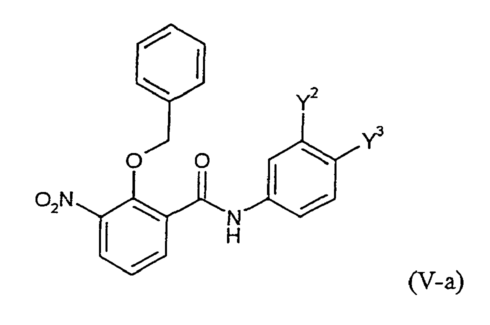

**Tabelle 7**

| Beispiel | Y² | Y³ | logP |
|---|---|---|---|
| (V-2) | -H | 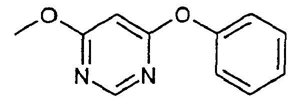 | 4,24 |
| (V-3) | -H | 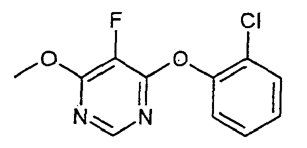 | 4,83 |
| (V-4) | -H | 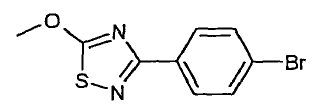 | 5,75 |
| (V-5) | -H | 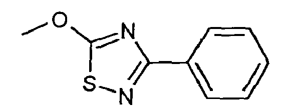 | 4,99 |
| (V-6) | -H | 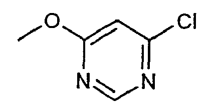 | 3,77 |
| (V-7) | -CH₃ | 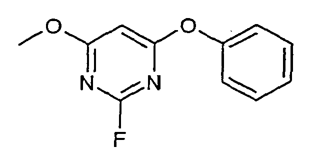 | 4,65 |
| (V-8) | -H | 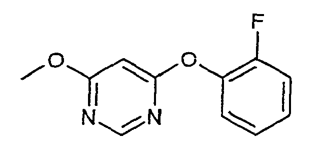 | 4,33 |
| (V-9) | -H | 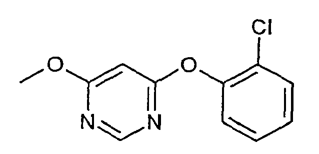 | 4,55 |
| (V-10) | -H | 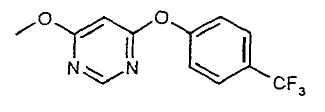 | 4,55 |
| (V-11) | -H | 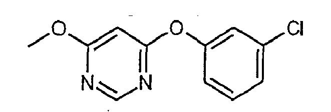 | 4,73 |
| (V-12) | -H | 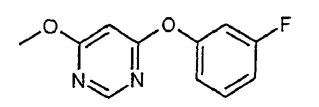 | 4,35 |
| (V-13) | -H | 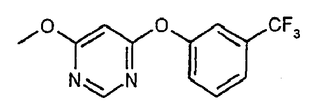 | 4,79 |
| (V-14) | -H | 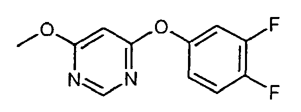 | 4,42 |
| (V-15) | -H | 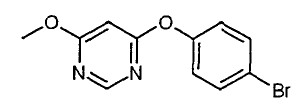 | 4,80 |
| (V-16) | -H | 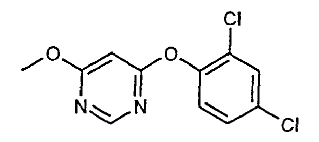 | 5,12 |
| (V-17) | -H | 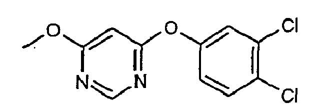 | 5,05 |
| (V-18) | -H | 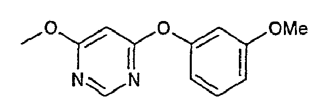 | 4,21 |
| (V-19) | -H | 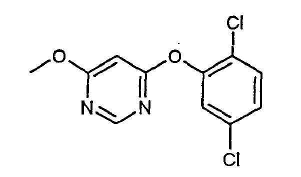 | 5,05 |
| (V-20) | -H | 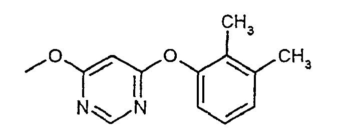 | 4,77 |
| (V-21) | -H | 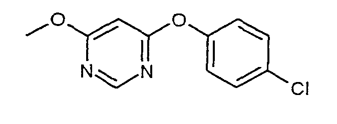 | 4,69 |
| (V-22) | -H | 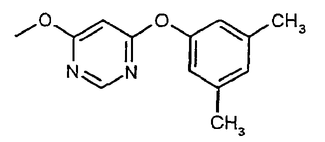 | 4,90 |
| (V-23) | -H | 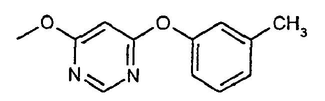 | 4,56 |
| (V-24) | -H | 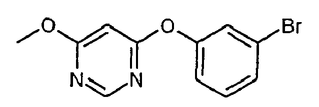 | 4,84 |
| (V-25) | -H | 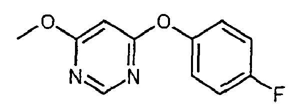 | 4,28 |
| (V-26) | -H | 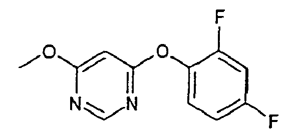 | 4,42 |
| (V-27) | -H | 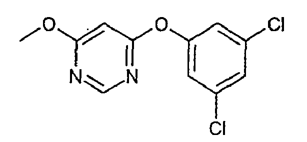 | 5,39 |
| (V-28) | -H | 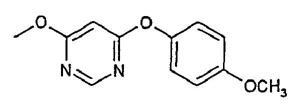 | 4,15 |
| (V-29) | -H | 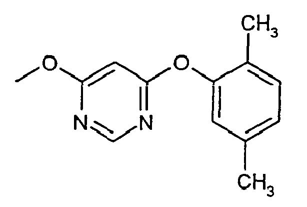 | 4,85 |
| (V-30) | -H | 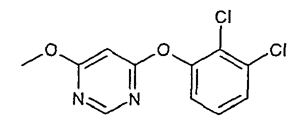 | 4,96 |
| (V-31) | -H | 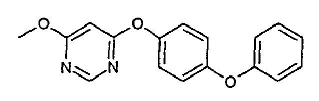 | 5,06 |
| (V-32) | -H | 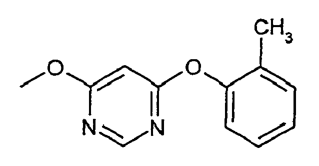 | 4,52 |
| (V-33) | -H | 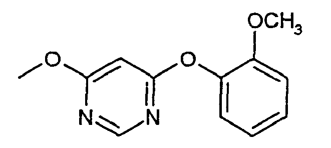 | 4,09 |
| (V-34) | -H | 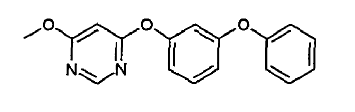 | 5,12 |

Analog Beispiel (VII-a-1) und (VII-a-2), sowie entsprechend der allgemeinen Beschreibung der erfindungsgemäßen Verfahren h) und i) wurden die in der nachfolgenden Tabelle 8 genannten Verbindungen der Formel (VII-b) erhalten:

**Tabelle 8**

| | | | | | | | |
|---|---|---|---|---|---|---|---|
| 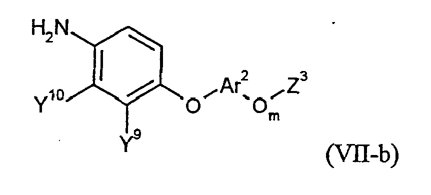 | | | | | | | |

| Bsp-Nr | Y¹⁰ | Y⁹ | Ar² | m | Z³ | Fp.: | LogP |
|---|---|---|---|---|---|---|---|
| VII-a-3 | -H | -H | 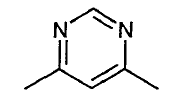 | 1 | Phenyl | 130 | 1,53 |
| VII-a-4 -H | | -H | 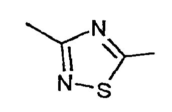 | 0 | Phenyl | | 2,57 |
| VH-a-5 | -H | -H | 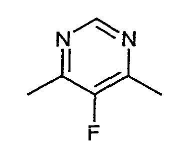 | 1 | Phenyl | | 1,88 |
| VII-a-6 | -H | -CH3 | 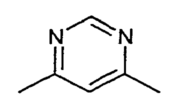 | 1 | Phenyl | | 2,32 |
| VH-a-7 | -H | -H | 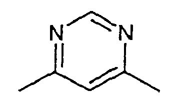 | 1 | 4-Chlor-phenyl | | 2,12 |
| VII-a-8 | -H | -H | 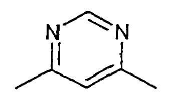 | 1 | 3,4-Difluor-phenyl | | 4,55 |
| VII-a-9 | -H | -H | 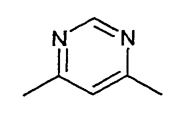 | 1 | 4-Brom-phenyl | | 2,02 |
| VII-a-10 | -H | -H | 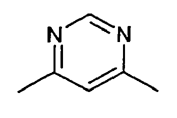 | 1 | 2,4-Dichlor-phenyl | | 2,33 |
| VII-a-11 | -H | -H | 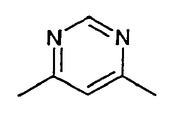 | 1 | 3,4-Dichlor-phenyl | | 2,48 |
| VII-a-12 | -H | -H | 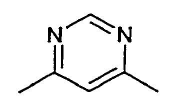 | 1 | 3-Methoxy-phenyl | | 1,61 |
| VII-a-13 | -H | -H | 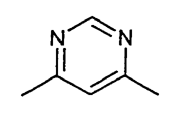 | 1 | 4-Tri-fluormethylphenyl | | 2,21 |
| VII-a-14 | -H | -H | 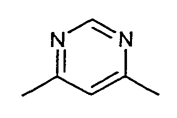 | 1 | 3-Trifluormethylphenyl | | 2,18 |
| VII-a-15 | -H | -H | 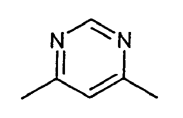 | 1 | 2-Fluor-phenyl | | 1,61 |
| VII-a-16 | -H | -H | 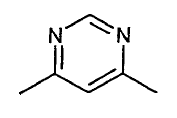 | 1 | 3-Fluor-phenyl | | 1,46 |
| VII-a-17 | -H | -H | 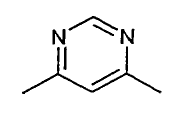 | 1 | 3-Tolyl | | 2,12 |
| VII-a-18 | -H | -H | 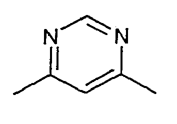 | 1 | 2,5-Dichlor-phenyl | | 2,26 |
| VII-a-19 | -H | -H | 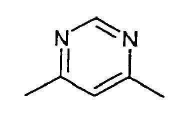 | 1 | 2,3-Di-methylphenyl | | 1,98 |
| VII-a-20 | -H | -H | 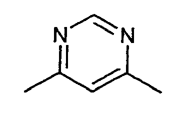 | 1 | 3,5-Di-methylphenyl | | 2,12 |
| VII-a-21 | -H | -H | 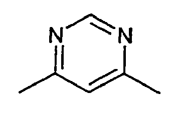 | 1 | 2,4-Difluor-phenyl | | 1,76 |
| VII-a-22 | -H | -H | 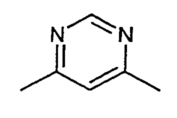 | 1 | 3,5-Dichlor-phenyl | | 2,6 |
| VII-a-23 | -H | -H | 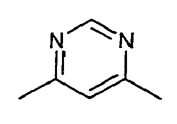 | 1 | 4-Methoxyphenyl | | 1,52 |
| VII-a-24 | -H | -H | 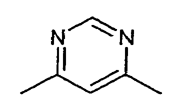 | 1 | 4-Fluor-phenyl | | 1,59 |
| VII-a-25 | -H | -H | 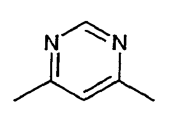 | 1 | 3-Brom-phenyl | | 2,05 |
| VII-a-26 | -H | -H | 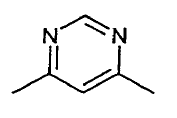 | 1 | 2,3-Dichlor-phenyl | | 2,26 |
| VII-a-27 | -H | -H | 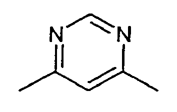 | 1 | 2,5-Di-methylphenyl | | 2,02 |
| VII-a-28 | -H | -H | 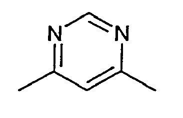 | 1 | 4-Phenoxy-phenyl | | 2,55 |
| VII-a-29 | -H | -H | 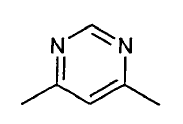 | 1 | 2-Methoxy-phenyl | | 1,46 |
| VII-a-30 | -H | -H | 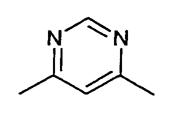 | 1 | 2-Tolyl | | 1,71 |
| VII-a-31 | -H | -H | 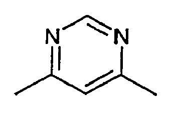 | 1 | 3-Phenoxy-phenyl | | 2,54 |
| VII-a-32 | -H | -H | 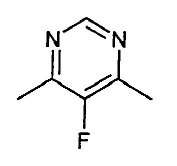 | 1 | 2-Chlor-phenyl | | 3,61 |
| VII-a-33 | -H | -H | 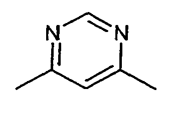 | 1 | 2,6-Di-methylphenyl | | |
| VII-a-34 | -H | -H | 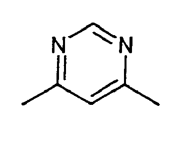 | 1 | 2,6-Dichlor-phenyl | | |

### Anwendungsbeispiele:

### Beispiel A

### Phytophthora-Test (Tomate) / protektiv

| | | |
|---|---|---|
| Lösungsmittel: | 47 | Gewichtsteile Aceton |
| Emulgator: | 3 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wäßrigen Sporensuspension von Phytophthora infestans inokuliert. Die Pflanzen werden dann in einer Inkubationskabine bei ca. 20°C und 100 % relativer Luftfeuchtigkeit aufgestellt.

3 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, daß kein Befall beobachtet wird.

Bei diesem Test zeigen z.B. die folgenden Verbindungen der Herstellungsbeispiele 1, 2 und 4 bei einer beispielhaften Wirkstoffaufwandmenge von 100 g/ha einen Wirkungsgrad von über 80% im Vergleich zur unbehandelten Kontrolle.

### Beispiel B

### Venturia-Test (Apfel) / protektiv

| | | |
|---|---|---|
| Lösungsmittel: | 47 | Gewichtsteile Aceton |
| Emulgator: | 3 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wäßrigen Konidiensuspension des Apfelschorferregers ***Venturia inaequalis*** inokuliert und verbleiben dann 1 Tag bei ca. 20°C und 100 % relativer Luftfeuchtigkeit in einer Inkubationskabine.

Die Pflanzen werden dann im Gewächshaus bei ca. 21°C und einer relativen Luftfeuchtigkeit von ca. 90 % aufgestellt.

12 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, daß kein Befall beobachtet wird.

Bei diesem Test zeigen z.B. die folgenden Verbindungen der Herstellungsbeispiele 1 und 2 bei einer beispielhaften Wirkstoffaufwandmenge von 100 g/ha einen Wirkungsgrad von über 80% im Vergleich zur unbehandelten Kontrolle.

## Patentansprüche

1. 4,6-Dichlor-5-fluorpyrimidin der Formel (XIII),

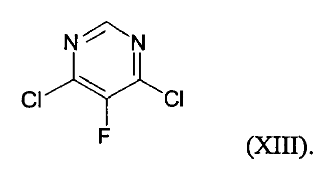

2. Verfahren zur Herstellung von 4,6-Dichlor-5-fluorpyrimidin, **dadurch gekennzeichnet, daß** man (Verfahren k) 5-Fluor-6-hydroxy-4(1H)-pyrimidinon (XIV) mit einem Chlorierungsmittel, gegebenenfalls in Gegenwart eines Verdünnungsmittels und gegebenenfalls in Gegenwart eines Katalysators, umsetzt.

3. Verbindungen der Formel 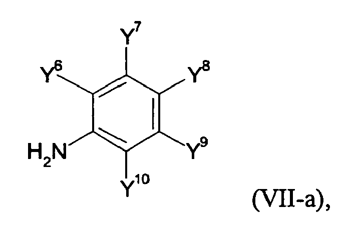 in welcher
Y⁶, Y⁷, Y⁸, Y⁹ und Y¹⁰ gleich oder verschieden sind und für Wasserstoff, Halogen, Cyano, Alkyl, Halogenalkyl, Alkoxy oder Halogenalkoxy stehen, und entweder
Y⁷ oder Y⁸ für eine Gruppierung 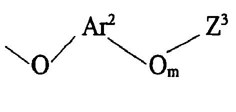 steht,
worin
m für 0 oder 1 steht,
Ar² für 1,2,4-Oxadiazol-3,5-diyl, 1,2,4-Thiadiazol-3,5-diyl oder für gegebenenfalls in 5-Stellung durch Halogen substituiertes Pyrimidin-4,6-diyl steht und
Z³ für gegebenenfalls substituiertes Aryl steht.

4. Verbindungen der Formel (VII-a) gemäß Anspruch 3,
in welcher
Y⁶, Y⁷, Y⁸, Y⁹ und Y¹⁰ gleich oder verschieden sind und für Wasserstoff, Halogen, Cyano, Alkyl oder Alkoxy mit jeweils 1 bis 4 Kohlenstoffatomen, Halogenalkyl, oder Halogenalkoxy mit jeweils 1 bis 4 Kohlenstoffatomen und 1 bis 5 gleichen oder verschiedenen Halogenatomen stehen, und entweder
Y⁷ oder Y⁸ für eine Gruppierung 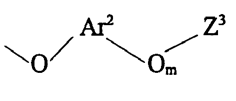 steht,
worin
m für 0 oder 1 steht,
Ar² für 1,2,4-Oxadiazol-3,5-diyl, 1,2,4-Thiadiazol-3,5-diyl oder für gegebenenfalls in 5-Stellung durch Fluor oder Chlor substituiertes Pyrimidin-4,6-diyl steht und
Z³ für jeweils gegebenenfalls einfach oder mehrfach, gleich oder verschieden substituiertes Phenyl oder Naphthyl steht, wobei die möglichen Substituenten vorzugsweise aus der nachstehenden Aufzählung ausgewählt sind:
Halogen, Cyano, Amino, Hydroxy, Formyl, Carboxy, Carbamoyl, Thiocarbamoyl;
jeweils geradkettiges oder verzweigtes Alkyl, Alkoxy, Alkylthio, Alkylsulfinyl oder Alkylsulfonyl mit jeweils 1 bis 6 Kohlenstoffatomen;
jeweils geradkettiges oder verzweigtes Alkenyl oder Alkenyloxy mit jeweils 2 bis 6 Kohlenstoffatomen;
jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen;
jeweils geradkettiges oder verzweigtes Halogenalkenyl oder Halogenalkenyloxy mit jeweils 2 bis 6 Kohlenstoffatomen und 1 bis 11 gleichen oder verschiedenen Halogenatomen;
jeweils geradkettiges oder verzweigtes Alkylamino, Dialkylamino, Alkylcarbonyl, Alkylcarbonyloxy, Alkoxycarbonyl oder Alkylsulfonyloxy, mit jeweils 1 bis 6 Kohlenstoffatomen in den einzelnen Alkylteilen;
jeweils gegebenenfalls einfach oder mehrfach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen substituiertes, jeweils zweifach verknüpftes Alkylen oder Dioxyalkylen mit jeweils 1 bis 6 Kohlenstoffatomen;
Cycloalkyl mit 3 bis 6 Kohlenstoffatomen;
jeweils gegebenenfalls einfach oder mehrfach, gleich oder verschieden durch Halogen und/oder geradkettiges oder verzweigtes Alkyl mit 1 bis 4 Kohlenstoffatomen substituiertes Phenyl oder Phenoxy;
Heterocyclyl oder Heterocyclyl-methyl mit jeweils 3 bis 7 Ringgliedern, von denen jeweils 1 bis 3 gleiche oder verschiedene Heteroatome sind - insbesondere Stickstoff, Sauerstoff und/oder Schwefel-, oder
eine Gruppierung 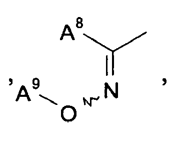 worin
A⁸ für Alkyl mit 1 bis 4 Kohlenstoffatomen oder Cycloalkyl mit 1 bis 6 Kohlenstoffatomen steht und
A⁹ für gegebenenfalls durch Cyano, Alkoxy, Alkylthio, Alkylamino, Dialkylamino oder Phenyl substituiertes Alkyl mit 1 bis 4 Kohlenstoffatomen, Alkenyl oder Alkinyl mit jeweils 2 bis 4 Kohlenstoffatomen steht, oder
eine Gruppierung , 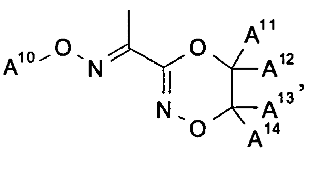 in welcher
A¹⁰ für Alkyl mit 1 bis 4 Kohlenstoffatomen steht und
A¹¹, A¹², A¹³ und A¹⁴ gleich oder verschieden sind und unabhängig voneinander für Wasserstoff, Alkyl oder Hydroxyalkyl mit jeweils 1 bis 4 Kohlenstoffatomen oder Halogenalkyl mit 1 bis 4 Kohlenstoffatomen und 1 bis fünf gleichen oder verschiedenen Halogenatomen stehen, oder
A¹¹ und A¹² oder A¹¹ und A¹³ oder A¹³ und A¹⁴ gemeinsam mit den jeweiligen Kohlenstoffatomen, an die sie gebunden sind, einen cycloaliphatischen Ring mit fünf, sechs oder sieben Kohlenstoffatomen bilden.

5. Verbindungen der Formel (VII-a) gemäß Anspruch 3,
in welcher
Y⁶, Y⁷, Y⁸, Y⁹ und Y¹⁰ gleich oder verschieden sind und für Wasserstoff, Fluor, Chlor, Brom, Cyano, Methyl, Ethyl, Methoxy, Trifluormethyl oder Difluormethoxy stehen, und entweder
Y⁷ oder Y⁸ für eine Gruppierung 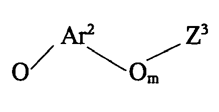 steht,
worin
m für 0 oder 1 steht,
Ar² für 1,2,4-Oxadiazol-3,5-diyl, 1,2,4-Thiadiazol-3,5-diyl oder für gegebenenfalls in 5-Stellung durch Fluor oder Chlor substituiertes Pyrimidin-4,6-diyl steht und
Z³ für jeweils gegebenenfalls einfach bis dreifach, gleich oder verschieden substituiertes Phenyl steht, wobei die möglichen Substituenten vorzugsweise aus der nachstehenden Aufzählung ausgewählt sind:
Fluor, Chlor, Brom, Cyano, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Trifluormethylthio, Difluorchlormethylthio, Trifluormethylsulfinyl oder Trifluormethylsulfonyl, Methoxycarbonyl, Ethoxycarbonyl,
jeweils gegebenenfalls einfach bis vierfach, gleich oder verschieden durch Fluor, Chlor, Methyl, Trifluormethyl oder Ethyl substituiertes, jeweils zweifach verknüpftes Methylendioxy, Ethylendioxy
Phenyl, 4-Chlorphenyl, 4-Methylphenyl, Phenoxy, 4- Chlorphenoxy, 4-Methylphenoxy, Cyclopropyl, Cyclobutyl, Cyclopentyl oder Cyclohexyl, oder
eine Gruppierung , 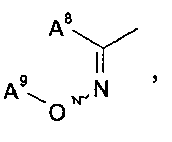 worin
A⁸ für Methyl, Ethyl, n- oder i-Propyl, n-, i-, s- oder t- Butyl, Cyclopropyl oder Cyclobutyl steht,
A⁹ für Methyl, Ethyl, n- oder i-Propyl, n-, i-, s- oder t- Butyl, Allyl, Propargyl, But-2-en-1-yl, 2-Methyl-prop- 1-en-3-yl, Cyanmethyl, Methoxymethyl, Ethoxymethyl, Methoxyethyl, Ethoxyethyl, Methylthiomethyl, Ethylthiomethyl, Methylthioethyl, Ethylthioethyl, Dimethylaminomethyl, Dimethylaminoethyl, Methylaminomethyl, Methylaminoethyl oder Benzyl steht, oder
eine Gruppierung , 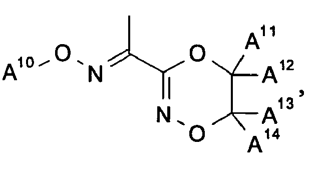 in welcher
A¹⁰ für Methyl oder Ethyl steht und
A¹¹, A¹², A¹³ und A¹⁴ gleich oder verschieden sind und unabhängig voneinander für Wasserstoff, Methyl, Ethyl, n- oder i-Propyl, n-, i-, s- oder t-Butyl, Hydroxymethyl, Trifluormethyl oder Trifluorethyl stehen, oder
A¹¹ und A¹² oder A¹¹ und A¹³ oder A¹³ und A¹⁴ gemeinsam mit den jeweiligen Kohlenstoffatomen, an die sie gebunden sind, einen cycloaliphatischen Ring mit fünf, sechs oder sieben Kohlenstoffatomen bilden.

6. Verfahren zur Herstellung von Verbindungen der Formel (VII-a) wie in Anspruch 3 definiert, **dadurch gekennzeichnet, daß** man Diazole der allgemeinen Formel (VIII) oder Halogenpyrimidine der allgemeinen Formel (IX), 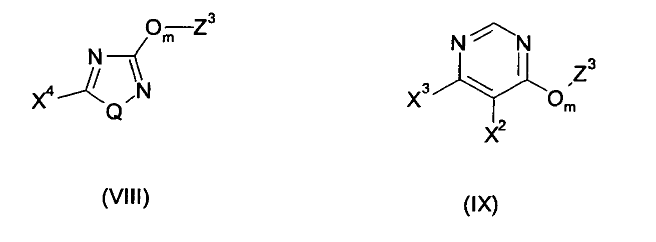 worin
m für 0 oder 1 steht,
X² für Wasserstoff Fluor oder Chlor steht,
X³ für Chlor oder Fluor steht,
X⁴ für Methylsulfonyl, Chlor oder Brom steht und
Q für Sauerstoff oder Schwefel steht, und
Z³ die in Anspruch 3 angegebene Bedeutung hat,
mit einem Aminophenol der Formel, 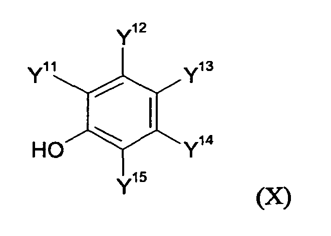 in welcher
Y¹¹, Y¹², Y¹³,Y¹⁴ und Y¹⁵gleich oder verschieden sind und für Wasserstoff, Halogen, Cyano, Alkyl, Halogenalkyl, Alkoxy oder Halogenalkoxy stehen, und entweder
Y¹² oder Y¹³ für Amino steht,
gegebenenfalls in Gegenwart eines Verdünnungsmittels, gegebenenfalls in Gegenwart eines Säureakzeptors und gegebenenfalls in Gegenwart eines Katalysators, umsetzt, oder
(Verfahren i) Aminophenoxypyrimidine der allgemeinen Formel (XI), 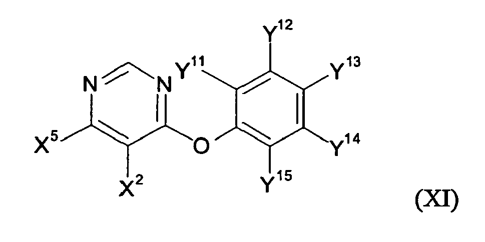 in welcher
X², Y¹², Y¹², Y¹³, Y¹⁴ und Y¹⁵ die oben angegebenen Bedeutungen haben und
X⁵ für Chlor oder Fluor steht,
mit einem Phenol der allgemeinen Formel (XII),
Z³-OH (XII)
in welcher
Z³ die oben angegebene Bedeutung hat,
gegebenenfalls in Gegenwart eines Verdünnungsmittels, gegebenenfalls in Gegenwart eines Säureakzeptors und gegebenenfalls in Gegenwart eines Katalysators, umsetzt.

7. Verbindungen der Formel (IX-a) 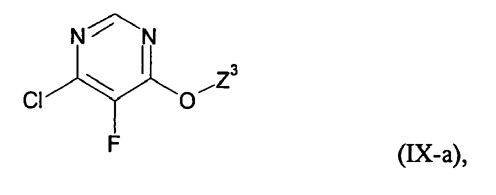 in welcher
Z³ die in den Ansprüchen 3 bis 5 angegebenen Bedeutungen hat, wobei die Verbindung 2-[(6-Chlor-5-fluor-4-pyrimidinyl)-oxy]-α-(methoxy- methylen)-benzoesäuremethylester, ausgenommen ist.

8. Verfahren zur Herstellung von Verbindungen der Formel (IX-a) wie in Anspruch 7, definiert, **dadurch gekennzeichnet, daß** man (Verfahren j) Phenole der allgemeinen Formel (XII),
Z³-OH (XII)
in welcher
Z³ die in den Ansprüchen 3 bis 5 angegebenen Bedeutungen hat,
mit 4,6-Dichlor-5-fluorpyrimidin (XIII), gegebenenfalls in Gegenwart eines Verdünnungsmittels, gegebenenfalls in Gegenwart eines Säureakzeptors und gegebenenfalls in Gegenwart eines Katalysators, umsetzt.

9. Verbindungen der Formel (XI), 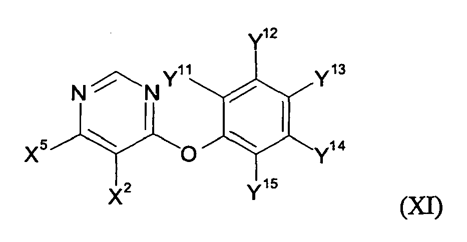 in welcher
X², X⁵, Y¹¹, Y¹², Y¹³, Y¹⁴ und Y¹⁵ die in Anspruch 6 angegebenen Bedeutungen haben.

10. Verfahren zur Herstellung von Verbindungen der Formel (XI) wie in Anspruch 9 definiert, **dadurch gekennzeichnet, daß** man (Verfahren 1) Trihalogenpyrimidine der Formel (XV), 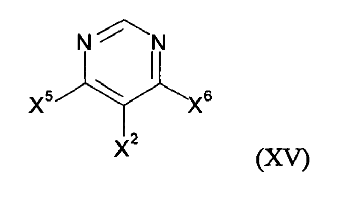 in welcher
X² und X⁵ die in Anspruch 6 angegebenen Bedeutungen haben und
X⁶ für Fluor oder Chlor steht,
mit einem Aminophenol der Formel (X),
gegebenenfalls in Gegenwart eines Verdünnungsmittels, gegebenenfalls in Gegenwart eines Säureakzeptors und gegebenenfalls in Gegenwart eines Katalysators, umsetzt.
